# EUROPEAN PATENT APPLICATION

(11) **EP 0 955 363 A2**
(43) Date of publication of application: **10.11.1999**
(21) Application number: 99107413.9
(22) Date of filing: 26.04.1999
(51) Int. Cl.: C12N 9/88, C12N 9/04, C12N 9/02, C12N 9/12, C12N 9/10, C12N 15/53, C12N 15/54, C12N 15/60, C12P 17/02

(54) **Dna sequences encoding enzymes involved in production of isoprenoids**

(30) Priority: 06.05.1998 EP 98108210
(71) Applicant: F. HOFFMANN-LA ROCHE AG, 4070 Basel (CH)
(72) Inventor: Hoshino, Tatsuo, Kamakura-shi, Kanagawa-ken 248-0027 (JP); Ojima, Kazuyuki, Fujisawa-shi, Kanagawa-ken 251-0877 (JP); Setoguchi, Yutaka, Fujisawa-shi, Kanagawa-ken 251-0033 (JP)
(74) Representative: Braun, Axel

(57) **Abstract**

The present invention is directed to an isolated DNA sequence coding for an enzyme involved in the mevalonate pathway or the pathway from isopentenyl pyrophosphate to farnesyl pyrophosphate, vectors or plasmids comprising such DNA, hosts transformed by either such DNAs or vectors or plasmids and a process for the production of isoprenoids and carotenoids by using such transformed host cells.

## Description

The present invention relates to molecular biology for the manufacture of isoprenoids and biological materials useful therefor.

Astaxanthin is known to distribute in a wide variety of organisms such as animal (e.g. birds such as flamingo and scarlet ibis, and fish such as rainbow trout and salmon), algae and microorganisms. It is also recognized that astaxanthin has a strong antioxidation property against oxygen radical, which is expected to apply to pharmaceutical usage to protect living cells against some diseases such as a cancer. Moreover, from a viewpoint of industrial application, a demand for astaxanthin as a coloring reagent is increasing especially in the industry of farmed fish, such as salmon, because astaxanthin imparts distinctive orange-red coloration to the animals and contributes to consumer appeal in the marketplace.

*Phaffia rhodozyma* is known as a carotenogenic yeast strain which produces astaxanthin specifically. Different from the other carotenogenic yeast, *Rhodotorula* species, *Phaffia rhodozyma* (*P. rhodozyma*) can ferment some sugars such as D-glucose. This is an important feature from a viewpoint of industrial application. In a recent taxonomic study, a sexual cycle of *P. rhodozyma* was revealed and its telemorphic state was designated under the name of *Xanthophyllomyces dendrorhous* (W.I. Golubev; Yeast 11, 101 - 110, 1995). Some strain improvement studies to obtain hyper producers of astaxanthin from *P. rhodozyma* have been conducted, but such efforts have been restricted to employ the method of conventional mutagenesis and protoplast fusion in this decade. Recently, Wery *et al*. developed a host vector system using *P. rhodozyma* in which a non-replicable plasmid was used to be integrated onto the genome of *P. rhodozyma* at the locus of ribosomal DNA in multicopies (Wery *et al*., Gene, 184, 89-97, 1997). And Verdoes *et al*. reported more improved vectors to obtain a transformant of *P. rhodozyma* as well as its three carotenogenic genes which code the enzymes that catalyzes the reactions from geranylgeranyl pyrophosphate to β-carotene (International patent WO97/23633). The importance of genetic engineering method on the strain improvement study of *P. rhodozyma* will increase in near future to break through the reached productivity by the conventional methods.

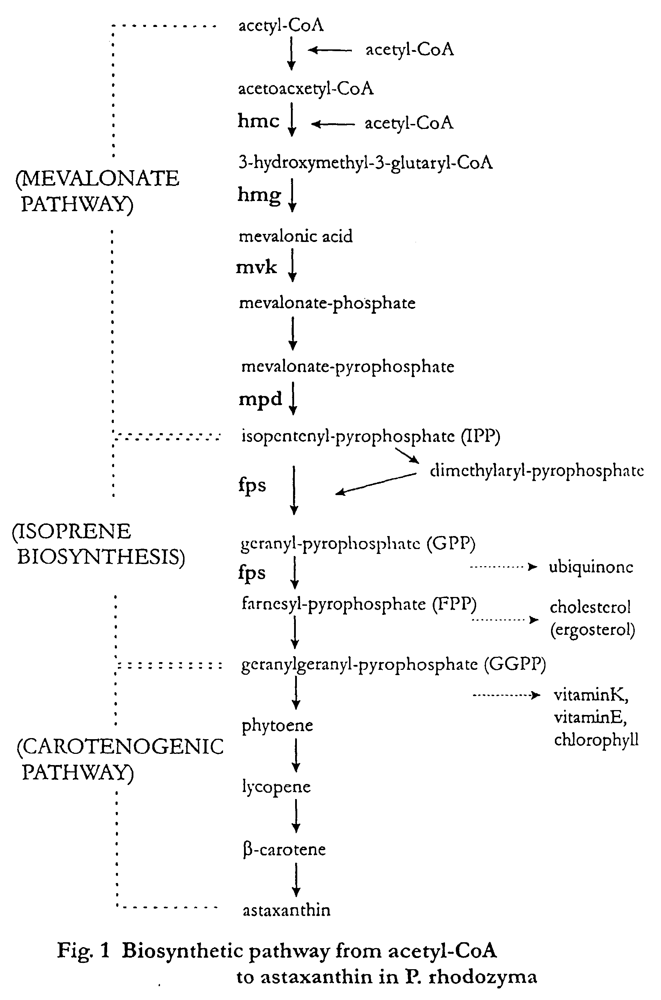

It is reported that the carotenogenic pathway from a general metabolite, acetyl-CoA consists of multiple enzymatic steps in carotenogenic eukaryotes as shown in Fig.1. Two molecules of acetyl-CoA are condensed to yield acetoacetyl-CoA which is converted to 3-hydroxy-3-methyglutaryl-CoA (HMG-CoA) by the action of 3-hydroxymethyl-3-glutaryl-CoA synthase. Next, 3-hydroxy-3-methylglutaryl-CoA reductase converts HMG-CoA to mevalonate, to which two molecules of phosphate residues are then added by the action of two kinases (mevalonate kinase and phosphomevalonate kinase). Mevalonate pyrophosphate is then decarboxylated by the action of mevalonate pyrophosphate decarboxylase to yield isopentenyl pyrophosphate (IPP) which becomes a building unit of wide varieties of isoprene molecules which is necessary in living organisms. This pathway is called as mevalonate pathway taken from its important intermediate, mevalonate. IPP is isomerized to dimethylaryl pyrophosphate (DMAPP) by the action of IPP isomerase. Then, IPP and DMAPP converted to C₁₀ unit, geranyl pyrophosphate (GPP) by the head to tail condensation. In a similar condensation reaction between GPP and IPP, GPP is converted to C₁₅ unit, farnesyl pyrophosphate (FPP) which is an important substrate of cholesterol in animal and ergosterol in yeast, and of farnesylation of regulation protein such as RAS protein. In general, the biosynthesis of GPP and FPP from IPP and DMAPP are catalyzed by one enzyme called FPP synthase (Laskovics *et al*., Biochemistry, 20, 1893-1901, 1981). On the other hand, in prokaryotes such as eubacteria, isopentenyl pyrophosphate was synthesized in a different pathway via 1-deoxyxylulose-5-phosphate from pyruvate which is absent in yeast and animal (Rohmer *et al*., Biochem. J., 295, 517-524, 1993). In exclusive studies of cholesterol biosynthesis, it was shown that rate-limiting steps of cholesterol metabolism were in the steps of this mevalonate pathway, especially in its early steps catalyzed by HMG-CoA synthase and HMG-CoA reductase. The inventors paid their attention to the fact that the biosynthetic pathways of cholesterol and carotenoid share their intermediate pathway from acetyl-CoA to FPP, and tried to improve the rate-limiting steps in the carotenogenic pathway which might exist in the steps of mevalonate pathway, especially in early mevalonate pathway such as the steps catalyzed by HMG-CoA synthase and HMG-CoA reductase so as to improve the productivity of carotenoids, especially astaxanthin.

This invention is created based on the above endeavor of the inventors. In accordance with this invention, the genes and the enzymes involved in the mevalonate pathway from acetyl-CoA to FPP which are biological materials useful in the improvement of the astaxanthin production process are provided. This invention involves cloning and determination of the genes which code for HMG-CoA synthase, HMG-CoA reductase, mevalonate kinase, mevalonate pyrophosphate decarboxylase and FPP synthase. This invention also involves the enzymatic characterization as a result of the expression of such genes in suitable host organisms such as *E. coli*. These genes may be amplified in a suitable host, such as *P. rhodozyma* and their effects on the carotenogenesis can be confirmed by the cultivation of such a transformant in an appropriate medium under an appropriate cultivation condition.

According to the present invention, there are provided an isolated DNA sequence coding for an enzyme involved in the mevalonate pathway or the reaction pathway from isopentenyl pyrophosphate to farnesyl pyrophosphate. More specifically, the said enzyme are those having an activity selected from the group consisting of 3-hydroxy-3-methylglutaryl-CoA synthase activity, 3-hydroxy-3-methylglutaryyl-CoA reductase activity, mevalonate kinase activity, mevalonate pyrophosphate decarboxylase activity and farnesyl pyrophosphate synthase.

The said isolated DNA sequence may be more specifically characterized in that (a) it codes for the said enzyme having an amino acid sequence selected from the group consisting of those described in SEQ ID NOs: 6, 7, 8, 9 and 10, or (b) it codes for a variant of the said enzyme selected from (i) an allelic variant, and (ii) an enzyme having one or more amino acid addition, insertion, deletion and/or substitution and having the stated enzyme activity. Particularly specified isolated DNA sequence mentioned above may be that which can be derived from a gene of *Phaffia rhodozyma* and is selected from (i) a DNA sequence represented in SEQ ID NOs: 1, 2, 4 or 5; (ii) an isocoding or an allelic variant for the DNA sequence represented in SEQ ID NOs: 1, 2, 4 or 5; and (iii) a derivative of a DNA sequence represented in SEQ ID NOs: 1, 2, 4 or 5 with addition, insertion, deletion and/or substitution of one or more nucleotide(s), and coding for a polypeptide having the said enzyme activity. Such derivatives can be made by recombinant means on the basis of the DNA sequences as disclosed herein by methods known in the state of the art and disclosed e.g. by Sambrook et al. (Molecular Cloning, Cold Spring Harbour Laboratory Press, New York, USA, second edition 1989). Amino acid exchanges in proteins and peptides which do not generally alter the activity are known in the state of the art and are described, for example, by H. Neurath and R. L. Hill in ÒThe ProteinsÓ (Academic Press, New York, 1979, see especially Figure 6, page 14). The most commonly occurring exchanges are: Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, as well as these in reverse.

The present invention also provides an isolated DNA sequence, which is selected from (i) a DNA sequence represented in SEQ ID NO: 3; (ii) an isocoding or an allelic variant for the DNA sequence represented in SEQ ID NO: 3; and (iii) a derivative of a DNA sequence represented in SEQ ID NO: 3 with addition, insertion, deletion and/or substitution of one or more nucleotide(s), and coding for a polypeptide having the mevalonate kinase activity.

Furthermore the present invention is directed to those DNA sequences as specified above and as disclosed, e.g. in the sequence listing as well as their complementary strands, or those which include these sequences, DNA sequences which hybridize under standard conditions with such sequences or fragments thereof and DNA sequences, which because of the degeneration of the genetic code, do not hybridize under standard conditions with such sequences but which code for polypeptides having exactly the same amino acid sequence.

"Standard conditions" for hybridization mean in this context the conditions which are generally used by a man skilled in the art to detect specific hybridization signals and which are described, e.g. by Sambrook et al., "Molecular Cloning" second edition, Cold Spring Harbour Laboratory Press 1989, New York, or preferably so called stringent hybridization and non-stringent washing conditions or more preferably so called stringent hybridization and stringent washing conditions a man skilled in the art is familiar with and which are described, e.g. in Sambrook et al. (s.a.). Furthermore DNA sequences which can be made by the polymerase chain reaction by using primers designed on the basis of the DNA sequences disclosed herein by methods known in the art are also an object of the present invention. It is understood that the DNA sequences of the present invention can also be made synthetically as described, e.g. in EP 747 483.

Further provided by the present invention is a recombinant DNA, preferably a vector and/or plasmid comprising a sequence coding for an enzyme functional in the mevalonate pathway or the reaction pathway from isopentenyl pyrophosphate to farnesyl pyrophosphate. The said recombinant DNA vector and/or plasmid may comprise the regulatory regions such as promoters and terminators as well as open reading frames of above named DNAs.

The present invention also provides the use of the said recombinant DNA, vector or plasmid, to transform a host organism. The recombinant organism obtained by use of the recombinant DNA is capable of overexpressing DNA sequence encoding an enzyme involved in the mevalonate pathway or the reaction pathway from isopentenyl pyrophosphate to farnesyl pyrophosphate. The host organism transformed with the recombinant DNA may be useful in the improvement of the production process of isoprenoids and carotenoids, in particular astaxanthin. Thus the present invention also provides such a recombinant organism/transformed host.

The present invention further provides a method for the production of isoprenoids or carotenoids, preferably carotenoids, which comprises cultivating thus obtained recombinant organism.

The present invention also relates to a method for producing an enzyme involed in the mevalonate pathway or the reaction pathway from isopentenyl pyrophosphate to farnesyl pyrophosphate, which comprises culturing a recombinant organism mentioned above, under a condition conductive to the production of said enzyme and relates also to the enzyme itself.

The present invention will be understood more easily on the basis of the enclosed figures and the more detailed explanations given below.
Fig. 1 depicts a scheme of deduced biosynthetic pathway from acetyl-CoA to astaxanthin in *P. rhodozyma*.

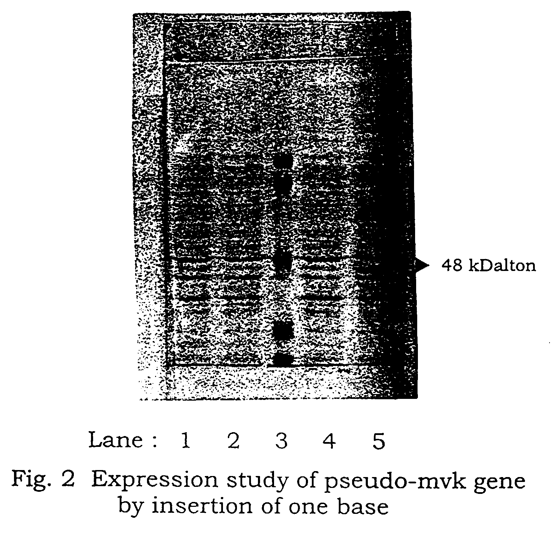

Fig. 2 shows the expression study by using an artificial *mvk* gene obtained from an artificial nucleotide addition at amino terminal end of pseudo-*mvk* gene from *P. rhodozyma*. The cells from 50 µl of broth were subjected to 10 % sodium dodesyl sulfide - polyacrylamide gel electrophoresis (SDS-PAGE). Lane 1, *E. coli* (M15 (pREP4) (pQE30) without IPTG); Lane 2, *E. coli* (M15 (pREP4) (pQE30) with 1mM IPTG); Lane 3, Molecular weight marker (105 kDa, 82.0 kDa, 49.0 kDa, 33.3 kD and 28.6 kDa, up to down, BIO-RAD); Lane 4, *E.coli* (M15 (pREP4) (pMK1209 #3334) without IPTG); Lane 5, *E.coli* (M15 (pREP4) (pMK1209 #3334) with 1mM IPTG).

The present invention provides an isolated DNA sequence which code for enzymes which are involved in a biological pathway comprising the mevalonate pathway or the reaction pathway from isopentenyl pyrophosphate to farnesyl pyrophosphate. The said enzymes can be exemplified by those involved in the mevalonate pathway or the reaction pathway from isopentenyl pyrophosphate to farnesyl pyrophosphate in *Phaffia rhodozyma*, such as 3-hydroxy-3-methylglutaryl-CoA synthase, 3-hydroxy-3-methylglutaryyl-CoA reductase, mevalonate kinase, mevalonate pyrophosphate decarboxylase and farnesyl pyrophosphate synthase. The present invention is useful for the production of the compounds involved in the biological pathway from the mevalonate pathway to the carotenogenic pathway and various products derived from such compounds. The compounds involved in the mevalonate pathway are acetoacetyl-CoA, 3-hydroxymethyl-3-glutaryl-CoA, mevalonic acid, mevalonate-phosphate, mevalonate-pyrophosphate and isopentenyl-pyrohposphate. Subsequently, isopentenyl-pyrohposphate is converted to geranylgeranyl-pyrophosphate through geranyl-pyrophosphate and farnesyl-pyrophosphate via the 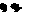 Isoprene Biosynthesis" reactions as indicated in Fig. 1. The compounds involved in the carotenogenic pathway are geranylgeranyl-pyrophosphate, phytoene, lycopene, β-carotene and astaxanthin. Among the compounds involved in the above-mentioned biosynthesis, geranyl-pyrophosphate may be utilized for the production of ubiquinone. Farnesyl-pyrophosphate can be utilized for the production of sterols, such as cholesterol and ergosterol. Geranylgeranyl-pyrophosphate is a useful material for the production of vitamin K, vitamin E, chlorophyll and the like. Thus the present invention will be particularly useful when it is applied to a biological production of isoprenoids. Isoprenoids is the general term which collectively designates a series of compounds having isopentenyl-pyrophosphate as a skeleton unit. Further examples of isoprenoids are vitamin A and vitamin D₃.

The said DNA of the present inveiton can mean a cDNA which contains only open reading frame flanked between the short fragments in its 5'- and 3'- untranslated region and a genomic DNA which also contains its regulatory sequences such as its promoter and terminator which are necessary for the expression of the gene of interest.

In general, the gene consists of several parts which have different functions from each other. In eukaryotes, genes which encode corresponding protein are transcribed to premature messenger RNA (pre-mRNA) differing from the genes for ribosomal RNA (rRNA), small nuclear RNA (snRNA) and transfer RNA (tRNA). Although RNA polymerase II (PolII) plays a central role in this transcription event, PolII can not solely start transcription without *cis* element covering an upstream region containing a promoter and an upstream activation sequence (UAS), and a *trans*-acting protein factor. At first, a transcription initiation complex which consists of several basic protein components recognize the promoter sequence in the 5'-adjacent region of the gene to be expressed. In this event, some additional participants are required in the case of the gene which is expressed under some specific regulation, such as a heat shock response, or adaptation to a nutrition starvation, and so on. In such a case, a UAS is required to exist in the 5'-untranslated upstream region around the promoter sequence, and some positive or negative regulator proteins recognize and bind to the UAS. The strength of the binding of transcription initiation complex to the promoter sequence is affected by such a binding of the *trans*-acting factor around the promoter, and this enables the regulation of the transcription activity.

After the activation of a transcription initiation complex by the phosphorylation, a transcription initiation complex initiates transcription from the transcription start site. Some parts of the transcription initiation complex are detached as an elongation complex from the promoter region to the 3' direction of the gene (this step is called as a promoter clearance event) and an elongation complex continues the transcription until it reaches to a termination sequence that is located in the 3'-adjacent downstream region of the gene. Pre-mRNA thus generated is modified in nucleus by the addition of cap structure at the cap site which almost corresponds to the transcription start site, and by the addition of polyA stretches at the polyA signal which locates at the 3'-adjacent downstream region. Next, intron structures are removed from coding region and exon parts are combined to yield an open reading frame whose sequence corresponds to the primary amino acid sequence of a corresponding protein. This modification in which a mature mRNA is generated is necessary for a stable gene expression. cDNA in general terms corresponds to the DNA sequence which is reverse-transcribed from this mature mRNA sequence. It can be synthesized by the reverse transcriptase derived from viral species by using a mature mRNA as a template, experimentally.

To express a gene which was derived from eukaryote, a procedure in which cDNA is cloned into an expression vector in *E. coli* is often used as shown in this invention. This causes from a fact that a specificity of intron structure varies among the organisms and an inability to recognize the intron sequence from other species. In fact, prokaryote has no intron structure in its own genetic background. Even in the yeast, genetic background is different between ascomycetea to which *Saccharomyces cerevisiae* belongs and basidiomycetea to which *P. rhodozyma* belongs. Wery *et al*. showed that the intron structure of actin gene from *P. rhodozyma* cannot be recognized nor spliced by the ascomycetous yeast, *Saccharomyces cerevisiae* (Yeast, 12, 641-651, 1996).

Some other researchers reported that intron structures of some kinds of the genes involve regulation of their gene expressions (Dabeva, M. D. *et al*., Proc. Natl. Acad. Sci. U.S.A., 83, 5854, 1986). It might be important to use a genomic fragment which has its introns in a case of self-cloning of the gene of a interest whose intron structure involves such a regulation of its own gene expression.

To apply a genetic engineering method for a strain improvement study, it is necessary to study its genetic mechanism in the event such as transcription and translation. It is important to determine a genetic sequence such as its UAS, promoter, intron structure and terminator to study the genetic mechanism.

According to this invention, the genes which code for the enzymes involving the mevalonate pathway were cloned from genomic DNA of *P. rhodozyma*, and their genomic sequence containing HMG-CoA synthase (*hmc*) gene, HMG-CoA reductase (*hmg*) gene, mevalonate kinase (*mvk*) gene, mevalonate pyrophosphate decarboxylase (*mpd*) gene and FPP synthase (*fps*) gene including their 5'- and 3'-adjacent regions as well as their intron structures were determined.

At first, we cloned a partial gene fragment containing a portion of *hmc* gene, *hmg* gene, *mvk* gene, *mpd* gene and *fps* gene by using degenerate PCR method. The said degenerate PCR is a method to clone a gene of interest which has high homology of amino acid sequence to the known enzyme from other species which has a same or similar function. Degenerate primer, which is used as a primer in degenerate PCR, was designed by a reverse translation of the amino acid sequence to corresponding nucleotides ( 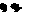 degenerated"). In such a degenerate primer, a mixed primer which consists any of A, C, G or T, or a primer containing inosine at an ambiguity code is generally used. In this invention, such the mixed primers were used for degenerate primers to clone above genes. PCR condition used is varied depending on primers and genes to clone as described hereinafter.

An entire gene containing its coding region with its intron as well as its regulation region such as a promoter or terminator can be cloned from a chromosome by screening of genomic library which is constructed in phage vector or plasmid vector in an appropriate host, by using a partial DNA fragment obtained by degenerate PCR as described above as a probe after it was labeled. Generally, *E. coli* as a host strain and *E. coli* vector, a phage vector such as λ phage vector, or a plasmid vector such as pUC vector is often used in the construction of library and a following genetic manipulation such as a sequencing, a restriction digestion, a ligation and the like. In this invention, an *EcoR*I genomic library of *P. rhodozyma* was constructed in the derivatives of λ vector, λZAPII and λDASHII depending on an insert size. An insert size, what length of insert must be cloned, was determined by the Southern blot hybridization for each gene before a construction of a library. In this invention, a DNA which was used for a probe was labeled with digoxigenin (DIG), a steroid hapten instead of conventional ³²P label, following the protocol which was prepared by the supplier (Boehringer-Mannheim). A genomic library constructed from the chromosome of *P. rhodozyma* was screened by using a DIG-labeled DNA fragment which had a portion of a gene of interest as a probe. Hybridized plaques were picked up and used for further study. In the case of using λDASHII (insert size was from 9 kb to 23 kb), prepared λDNA was digested by the *EcoR*I, followed by the cloning of the *EcoR*I insert into a plasmid vector such as pUC19 or pBluescriptII SK+. When λZAPII was used in the construction of the genomic library, *in vivo* excision protocol was conveniently used for the succeeding step of the cloning into the plasmid vector by using a derivative of single stranded M13 phage, Ex assist phage (Stratagene). A plasmid DNA thus obtained was examined for a sequencing.

In this invention, we used the automated fluorescent DNA sequencer, ALFred system (Pharmacia) using an autocycle sequencing protocol in which the Taq DNA polymerase is employed in most cases of sequencing.

After the determination of the genomic sequence, a sequence of a coding region was used for a cloning of cDNA of corresponding gene. The PCR method was also exploited to clone cDNA fragment. The PCR primers whose sequences were identical to the sequence at the 5'- and 3'- end of the open reading frame (ORF) were synthesized with an addition of an appropriate restriction site, and PCR was performed by using those PCR primers. In this invention, a cDNA pool was used as a template in this PCR cloning of cDNA. The said cDNA pool consists of various cDNA species which were synthesized *in vitro* by the viral reverse transcriptase and Taq polymerase (CapFinder Kit manufactured by Clontech was used) by using the mRNA obtained from *P. rhodozyma* as a template. cDNA of interest thus obtained was confirmed in its sequence. Furthermore, cDNA thus obtained was used for a confirmation of its enzyme activity after the cloning of the cDNA fragment into an expression vector which functions in *E. coli* under the strong promoter activity such as the *lac* or T7 expression system.

Succeeding to the confirmation of the enzyme activity, an expressed protein would be purified and used for raising of the antibody against the purified enzyme. Antibody thus prepared would be used for a characterization of the expression of the corresponding enzyme in a strain improvement study, an optimization study of the culture condition, and the like.

After the rate-limiting step is determined in the biosynthetic pathway which consists of multiple steps of enzymatic reactions, there are three strategies to enhance its enzymatic activity of the rate-limiting reaction by using its genomic sequence.

One strategy is to use its gene itself as a native form. The simplest approach is to amplify the genomic sequence including its regulation sequence such as a promoter and a terminator. This is realized by the cloning of the genomic fragment encoding the enzyme of interest into the appropriate vector on which a selectable marker that functions in *P. rhodozyma* is harbored. A drug resistance gene which encodes the enzyme that enables the host survive in the presence of a toxic antibiotic is often used for the selectable marker. G418 resistance gene harbored in pGB-Ph9 (Wery *et al*. (Gene, 184, 89-97, 1997)) is an example of a drug resistance gene. Nutrition complementation maker can be also used in the host which has an appropriate auxotrophy marker. *P. rhodozyma* ATCC24221 strain which requires cytidine for its growth is one example of the auxotroph. By using CTP synthetase as donor DNA for ATCC24221, a host vector system using a nutrition complementation can be established. As a vector, two types of vectors would be used. One of the vectors is an integrated vector which does not have an autonomous replicating sequence. Above pGB-Ph9 is an example of this type of a vector. Because such a vector does not have an autonomous replicating sequence in the vector, above vector cannot replicate by itself and can be present only in an integrated form on the chromosome of the host as a result of a single-crossing recombination using the homologous sequence between a vector and a chromosome. In case of increasing a dose of the integrated gene on the chromosome, amplification of the gene is often employed by using such a drug resistance marker. By increasing the concentration of the corresponding drug in the selection medium, the strain, in which the integrated gene is amplified on the chromosome as a result of recombination only can survive. By using such a selection, a strain which has amplified gene can be chosen. Another type of vector is a replicable vector which has an autonomous replicating sequence. Such a vector can exist in a multicopy state and this makes a dose of the harbored gene also exist in a multicopy state. By using such a strategy, an enzyme of interest which is coded by the amplified gene is expected to be overexpressed.

Another strategy to overexpress an enzyme of interest is a placement of a gene of interest under a strong promoter. In such a strategy, a copy number of a gene is not necessary to be in a multicopy state. This strategy is also applied to overexpress a gene of interest under the appropriate promoter whose promoter activity is induced in an appropriate growth phase and an appropriate timing of cultivation. Production of astaxanthin accelerates in a late phase of the growth such as the case of production of a secondary metabolite. Thus, the expression of carotenogenic genes may be maximized in a late phase of growth. In such a phase, gene expression of most biosynthesis enzyme decreases. For example, by placing a gene, which is involved in the biosynthesis of a precursor of astaxanthin and whose expression is under the control of a vegetative promoter such as a gene which encodes an enzyme which involves in mevalonate pathway, in the downstream of the promoter of carotenogenic genes, all the genes which are involved in the biosynthesis of astaxanthin become synchronized in their timings and phases of expression.

Still another strategy to overexpress enzymes of interest is induction of the mutation in its regulatory elements. For this purpose, a kind of reporter gene such as β-galactosidase gene, luciferase gene, a gene coding a green fluorescent protein, and the like is inserted between the promoter and the terminator sequence of the gene of interest so that all the parts including promoter, terminator and the reporter gene are fused and function each other. Transformed *P. rhodozyma* in which the said reporter gene is introduced on the chromosome or on the vector would be mutagenized *in vivo* to induce mutation within the promoter region of the gene of interest. Mutation can be monitored by detecting the change of the activity coded by the reporter gene. If the mutation occurs in a *cis* element of the gene, mutation point would be determined by the rescue of the mutagenized gene and sequencing. The determined mutation would be introduced to the promoter region on the chromosome by the recombination between a native promoter sequence and a mutated sequence. In the same procedure, the mutation occurring in the gene which encodes a *trans*-acting factor can be also obtained. It would also affect the overexpression of the gene of interest.

A mutation can be also induced by an *in vitro* mutagenesis of a *cis* element in the promoter region. In this approach, a gene cassette, containing a reporter gene which is fused to a promoter region derived from a gene of interest at its 5'-end and a terminator region from a gene of interest at its 3'-end, is mutagenized and then introduced into *P. rhodozyma*. By detecting the difference of the activity of the reporter gene, an effective mutation would be screened. Such a mutation can be introduced in the sequence of the native promoter region on the chromosome by the same method as the case of an *in vivo* mutation approach.

As a donor DNA, a gene which encodes an enzyme of mevalonate pathway or FPP synthase could be introduced solely or co-introduced by harboring on plasmid vector. A coding sequence which is identical to its native sequence, as well as its allelic variant, a sequence which has one or more amino acid additions, deletions and/or substitutions can be used as far as its corresponding enzyme has the stated enzyme activity. And such a vector can be introduced into *P. rhodozyma* by transformation and a transformant can be selected by spreading the transformed cells on an appropriate selection medium such as YPD agar medium containing geneticin in the case of pGB-Ph9 as a vector or a minimal agar medium omitting cytidine in the case of using auxotroph ATCC24221 as a recipient.

Such a genetically engineered *P. rhodozyma* would be cultivated in an appropriate medium and evaluated in its productivity of astaxanthin. A hyper producer of astaxanthin thus selected would be confirmed in view of the relationship between its productivity and the level of gene or protein expression which is introduced by such a genetic engineering method.

### Examples

The following materials and methods were emploied in the Example described below:

### Strains

*P. rhodozyma* ATCC96594 (This strain has been redeposited on April 8, 1998 as a Budapest Treaty deposit under accession No. 74438).
*E. coli* DH5α: F⁻, φ80d, *lac*ZΔM15, Δ(*lac*ZYA-*arg*F)U169, *hsd* (r_{K}⁻, m_{K}⁺), *rec*A1, *end*A1, *deo*R, *thi*-1, *sup*E44, *gyr*A96, *rel*A1 (Toyobo)
*E. coli* XL1-Blue MRF': Δ(*mcr*A)183, Δ(*mcr*CB-*hsd*SMR-*mrr*)173, *end*A1, *sup*E44, *thi*-1, *rec*A1, *gyr*A96, *rel*A1, *lac*[F' *pro*AB, *lac*I^{q}ZΔM15, Tn10 (tet^{r})] (Stratagene)
*E. coli* SOLR: e14⁻(*mcr*A), Δ(*mcr*CB-*hsd*SMR-*mrr*)171, *sbc*C, recB, *rec*J, *umu*C :: Tn5(kan^{r}), *uvr*C, *lac*, *gyr*A96, *rel*A1, *thi*-1, *end*A1, λ^{R}, [F' *pro*AB, *lacl*^{q}Z ΔM15] Su⁻(nonsuppressing) (Stratagene, CA, USA)
*E. coli* XL1 MRA (P2): Δ(*mcr*A)183, Δ(*mcr*CB-*hsd*SMR-*mrr*)173, *end*A1, *sup*E44, *thi*-1, *gyr*A96, *rel*A1, *lac* (P2 lysogen) (Stratagene)
*E. coli* BL21 (DE3) (pLysS): *dcm*⁻, *omp*Tr_{B}⁻ m_{B}⁻ *lon*⁻ λ(DE3), pLysS (Stratagene)
*E. coli* M15 (pREP4) (QIAGEN) (Zamenhof P. J. *et al*., J. Bacteriol. 110, 171-178, 1972)
*E. coli* KB822: *pcn*B80, *zad* :: Tn10, Δ(*lac*U169), *hsd*R17, *end*A1, *thi*-1, *sup*E44
*E. coli* TOP10: F⁻, *mcr*A, Δ(*mrr*-*hsd*RMS-*mcr*BC), φ80, Δ*lac*Z M15, Δ*lac*X74, *rec*A1, *deo*R, *ara*D139, (*ara-leu*)7697, *gal*U, *gal*K, *rps*L (Str^{r}), *end*A1, *nup*G (Invitrogen)

### Vectors

λZAPII (Stratagene)
λDASHII (Stratagene)
pBluescriptII SK+(Stratagene)
pUC57 (MBI Fermentas)
pMOSBlue T-vector (Amersham)
pET4c (Stratagene)
pQE30 (QIAGEN)
pCR2.1TOPO (Invitrogen)

### Media

*P. rhodozyma* strain is maintained routinely in YPD medium (DIFCO). *E. coli* strain is maintained in LB medium (10 g Bacto-trypton, 5 g yeast extract (DIFCO) and 5 g NaCl per liter). NZY medium (5 g NaCl, 2 g MgSO₄-7H₂O, 5 g yeast extract (DIFCO), 10 g NZ amine type A (Sheffield) per liter) is used for λ phage propagation in a soft agar (0.7 % agar (WAKO)). When an agar medium was prepared, 1.5 % of agar (WAKO) was supplemented.

### Methods

General methods of molecular genetics were practiced according to Molecular cloning: a Laboratory Manual, 2nd Edition (Cold Spring Harbor Laboratory Press, 1989). Restriction enzymes and T4 DNA ligase were purchased from Takara Shuzo (Japan).

Isolation of a chromosomal DNA from *P. rhodozyma* was performed by using QIAGEN Genomic Kit (QIAGEN) following the protocol supplied by the manufacturer. Mini-prep of plasmid DNA from transformed *E. coli* was performed with the Automatic DNA isolation system (PI-50, Kurabo, Co. Ltd., Japan). Midi-prep of plasmid DNA from an *E. coli* transformant was performed by using QIAGEN column (QIAGEN). Isolation of λ DNA was performed by Wizard lambda preps DNA purification system (Promega) following the protocol of the manufacturer. A DNA fragment was isolated and purified from agarose by using QIAquick or QIAEX II (QIAGEN). Manipulation of λ phage derivatives was done according to the protocol of the manufacturer (Stratagene).

Isolation of total RNA from *P. rhodozyma* was performed by the phenol method using Isogen (Nippon Gene, Japan). mRNA was purified from total RNA thus obtained by using mRNA separation kit (Clontech). cDNA was synthesized by using CapFinder cDNA construction kit (Clontech).

*In vitro* packaging was performed by using Gigapack III gold packaging extract (Stratagene).

Polymerase chain reaction (PCR) is performed with the thermal cycler from Perkin Elmer model 2400. Each PCR condition is described in examples. PCR primers were purchased from a commercial supplier or synthesized with a DNA synthesizer (model 392, Applied Biosystems). Fluorescent DNA primers for DNA sequencing were purchased from Pharmacia. DNA sequencing was performed with the automated fluorescent DNA sequencer (ALFred, Pharmacia).

Competent cells of DH5α were purchased from Toyobo (Japan). Competent cells of M15 (pREP4) were prepared by CaCl₂ method as described by Sambrook *et al.* (Molecular cloning: a Laboratory Manual, 2nd Edition, Cold Spring Harbor Laboratory Press, 1989).

### Example 1 Isolation of mRNA from P. rhodozyma and construction of cDNA library

To construct cDNA library of *P. rhodozyma*, total RNA was isolated by phenol extraction method right after the cell disruption and the mRNA from *P. rhodozyma* ATCC96594 strain was purified by using mRNA separation kit (Clontech).

At first, Cells of ATCC96594 strain from 10 ml of two-day-culture in YPD medium were harvested by centrifugation (1500 x g for 10 min.) and washed once with extraction buffer (10 mM Na-citrate / HCl (pH 6.2) containing 0.7 M KCl). After suspending in 2.5 ml of extraction buffer, the cells were disrupted by French press homogenizer (Ohtake Works Corp., Japan) at 1500 kgf/cm² and immediately mixed with two times of volume of isogen (Nippon gene) according to the method specified by the manufacturer. In this step, 400 µg of total RNA was recovered.

Then this total RNA was purified by using mRNA separation kit (Clontech) according to the method specified by the manufacturer. Finally, 16 µg of mRNA from *P. rhodozyma* ATCC96594 strain was obtained.

To construct cDNA library, CapFinder PCR cDNA construction kit (Clontech) was used according to the method specified by the manufacturer. One µg of purified mRNA was applied for a first strand synthesis followed by PCR amplification. After this amplification by PCR, 1 mg of cDNA pool was obtained.

### Example 2 Cloning of the partial hmc (3-hydroxy-3-methylglutaryl-CoA synthase) gene from P. rhodozyma

To clone a partial *hmc* gene from *P. rhodozyma*, a degenarate PCR method was exploited. Two mixed primers whose nucleotide sequences were designed and synthsized as shown in TABLE 1 based on the common sequence of known HMG-CoA synthase genes from other species.

**TABLE 1**

| Sequence of primers used in the cloning of *hmc* gene |
|---|
| Hmgs1 ; GGNAARTAYACNATHGGNYTNGGNCA (sense primer) (SEQ ID NO: 11) |
| Hmgs3 ; TANARNSWNSWNGTRTACATRTTNCC (antisense primer) (SEQ ID NO: 12) |
| (N=A, C, G or T; R=A or G, Y=C or T, H=A, T or C, S=C or G, W=A or T) |

After the PCR reaction of 25 cycles of 95 °C for 30 seconds, 50 °C for 30 seconds and 72°C for 15 seconds by using ExTaq (Takara Shuzo) as a DNA polymerase and cDNA pool obtained in example 1 as a template, reaction mixture was applied to agarose gel electrophoresis. A PCR band that has a desired length was recovered and purified by QIAquick (QIAGEN) according to the method by the manufacturer and then ligated to pMOSBlue T-vector (Amersham). After the transformation of competent *E. coli* DH5α, 6 white colonies were selected and plasmids were isolated with Automatic DNA isolation system. As a result of sequencing, it was found that 1 clone had a sequence whose deduced amino acid sequence was similar to known *hmc* genes. This isolated cDNA clone was designated as pHMC211 and used for further study.

### Example 3 Isolation of genomic DNA from P. rhodozyma

To isolate a genomic DNA from *P. rhodozyma*, QIAGEN genomic kit was used according to the method specified by the manufacturer.

At first, cells of *P. rhodozyma* ATCC96594 strain from 100 ml of overnight culture in YPD medium were harvested by centrifugation (1500 x g for 10 min.) and washed once with TE buffer (10 mM Tris / HCl (pH 8.0) containing 1 mM EDTA). After suspending in 8 ml of Y1 buffer of the QIAGEN genomic kit, lyticase (SIGMA) was added at the concentration of 2 mg/ml to disrupt cells by enzymatic degradation and the reaction mixture was incubated for 90 minutes at 30 °C and then proceeded to the next extraction step. Finally, 20 µg of genomic DNA was obtained.

### Example 4 Southern blot hybridization by using pHMC211 as a probe

Southern blot hybridization was performed to clone a genomic fragment which contains *hmc* gene from *P. rhodozyma*. Two µg of genomic DNA was digested by *EcoR*I and subjected to agarose gel electrophoresis followed by acidic and alkaline treatment. The denatured DNA was transferred to nylon membrane (Hybond N+, Amersham) by using transblot (Joto Rika) for an hour. The DNA which was transferred to nylon membrane was fixed by a heat treatment (80 °C, 90 minutes). A probe was prepared by labeling a template DNA (*EcoR*I- and *Sal*I- digested pHMC211) with DIG multipriming method (Boehringer Mannheim). Hybridization was performed with the method specified by the manufacturer. As a result, hybridized band was visualized in the range from 3.5 to 4.0 kilobases (kb).

### Example 5 Cloning of a genomic fragment containing hmc gene

Four µg of the genomic DNA was digested by *EcoR*I and subjected to agarose gel electrophoresis. Then, DNAs whose length is within the range from 3.0 to 5.0 kb was recovered by QIAEX II gel extraction kit (QIAGEN) according to the method specified by the manufacturer. The purified DNA was ligated to 1 µg of *EcoR*I-digested and CIAP (calf intestine alkaline phosphatase) -treated λZAPII (Stratagene) at 16 °C overnight, and packaged by Gigapack III gold packaging extract (Stratagene). The packaged extract was infected to *E. coli* XL1Blue MRF' strain and over-laid with NZY medium poured onto LB agar medium. About 6000 plaques were screened by using *EcoR*I- and *Sal*I- digested pHMC211 as a probe. Two plaques were hybridized to the labeled probe and subjected to *in vivo* excision protocol according to the method specified by the manufacturer (Stratagene). It was found that isolated plasmids had the same fragments in the opposite direction each other as results of restriction analysis and sequencing. As a result of sequencing, the obtained *EcoR*I fragment contained same nucleotide sequence as that of pHMC211 clone. One of these plasmids was designated as pHMC526 and used for further study. A complete nucleotide sequence was obtained by sequencing of deletion derivatives of pHMC526, and sequencing with a primer-walking procedure. The insert fragment of pHMC526 consists of 3431 nucleotides that contained 10 complete and an incomplete exons and 10 introns with about 1 kb of 3'-terminal untranslated region.

### Example 6 Cloning of upstream region of hmc gene

Cloning of 5'- adjacent region of *hmc* gene was performed by using Genome Walker Kit (Clontech), because pHMC 526 does not contain its 5' end of *hmc* gene. At first, the PCR primers whose sequences were shown in TABLE 2 were synthesized.

**TABLE 2**

| Sequence of primers used in the cloning of 5'- adjacent region of *hmc* gene |
|---|
| Hmc21 ; GAAGAACCCCATCAAAAGCCTCGA (primary primer) (SEQ ID NO: 13) |
| Hmc22 ; AAAAGCCTCGAGATCCTTGTGAGCG (nested primer) (SEQ ID NO: 14) |

Protocols for library construction and PCR condition were the same as those specified by the manufacturer by using the genomic DNA preparation obtained in Example 3 as a PCR template. The PCR fragments that had *EcoR*V site at the 5' end (0.45 kb), and that had *Pvu*II site at the 5' end (2.7 kb) were recovered and cloned into pMOSBlue T-vector by using *E. coli* DH5α as a host strain. As a result of sequencing of each 5 of independent clones from both constructs, it was confirmed that the 5' adjacent region of *hmc* gene was cloned and small part (0.1 kb) of *EcoR*I fragment within its 3' end was found. The clone obtained by the *Pvu*II construct in the above experiment was designated as pHMCPv708 and used for further study.

Next, Southern blot analysis was performed by the method as shown in above Example 4, and 5'- adjacent region of the *hmc* gene existed in 3 kb of *EcoR*I fragment was determined. After a construction from 2.5 to 3.5 kb *EcoR*I library in λZAPII, 600 plaques were screened and 6 positive clones were selected. As a result of sequencing of these 6 clones, it was clarified that 4 clones within 6 positive plaques had the same sequence as that of the pHMCPv708, and one of those was named as pHMC723 and used for further analysis.

The PCR primers whose sequences were shown in TABLE 3 were synthesized to clone small (0.1 kb) *EcoR*I fragment locating between 3.5 kb and 3.0 kb *EcoR*I fragments on the chromosome of *P. rhodozyma*.

**TABLE 3**

| Sequence of primers used in the cloning small *EcoR*I portion of *hmc* gene. |
|---|
| Hmc30 ; AGAAGCCAGAAGAGAAAA (sense primer) (SEQ ID NO: 15) |
| Hmc31 ; TCGTCGAGGAAAGTAGAT (antisense primer) (SEQ ID NO: 16) |

The PCR condition was the same as shown in Example 2. Amplified fragment (0.1 kb in its length) was cloned into pMOSBlue T-vector and transformed *E. coli* DH5α. Plasmids were prepared from 5 independent white colonies and subjected to the sequencing.

Thus, it was determined that the nucleotide sequence (4.8 kb) contained *hmc* gene (SEQ ID NO: 1). Coding region was in 2432 base pairs that consisted of 11 exons and 10 introns. Introns were scattered all through the coding region without 5' or 3' bias. It was found that open reading frame consists of 467 amino acids (SEQ ID NO: 6) whose sequence is strikingly similar to the known amino acid sequence of HMG-CoA synthase gene from other species (49.6 % identity to HMG-CoA synthase from *Schizosaccharomyces pombe*).

### Example 7 Expression of hmc gene in E. coli and confirmation of its enzymatic activity

The PCR primers whose sequences were shown in TABLE 4 were synthesized to clone a cDNA fragment of *hmc* gene.

**TABLE 4**

| Sequence of primers used in the cloning of cDNA of *hmc* gene |
|---|
| Hmc25 ; GGTACCATATGTATCCTTCTACTACCGAAC (sense primer) (SEQ ID NO: 17) |
| Hmc26 ; GCATGCGGATCCTCAAGCAGAAGGGACCTG (antisense primer) (SEQ ID NO: 18) |

PCR condition was as follows; 25 cycles of 95 °C for 30 seconds, 55 °C for 30 seconds and 72 °C for 3 minutes. As a template, 0.1 µg of cDNA pool obtained in Example 2 was used, and Pfu polymerase as a DNA polymerase. Amplified 1.5 kb fragment was recovered and cloned in pT7Blue-3 vector (Novagen) by using perfectly blunt cloning kit (Novagen) according to the protocol specified by the manufacturer. Six independent clones from white colonies of *E. coli* DH5α transformants were selected and plasmids were prepared from those transformants. As a result of restriction analysis, 2 clones were selected for a further selection by sequencing. One clone has an amino acid substitution at position 280 (from glycine to alanine) and another has at position 53 (from alanine to threonine). Alignment of an amino acid sequences derived from known *hmc* genes showed that alanine residue as well as glycine residue at position 280 was observed well in all the sequences from other species and this fact suggested that amino acid substitution at position 280 would not affect its enzymatic activity. This clone (mutant at position 280) was selected as pHMC731 for a succeeding expression experiment.

Next, 1.5 kb fragment obtained by *Nde*I- and *BamH*I- digestion of pHMC731 was ligated to pET11c (Stratagene) digested by the same pairs of restriction enzymes, and introduced to *E. coli* DH5α. As a result of restriction analysis, plasmid that had a correct structure (pHMC818) was recovered. Then, competent *E. coli* BL21 (DE3) (pLysS) cells (Stratagene) were transformed, and one clone that had a correct structure was selected for further study.

For an expression study, strain BL21 (DE3) (pLysS) (pHMC818) and vector control strain BL21 (DE3) (pLysS) (pET11c) were cultivated in 100 ml of LB medium at 37 °C until OD at 600 nm reached to 0.8 (about 3 hours) in the presence of 100 µg/ml of ampicillin. Then, the broth was divided in two portions of the same volume, and then 1 mM of isopropyl β-D-thiogalactopyranoside (IPTG) was added to one portion. Cultivation was continued for further 4 hours at 37 °C. Twenty five µl of broth was removed from induced- and uninduced- culture of *hmc* clone and vector control cultures and subjected to sodium dodesyl sulfate - polyacrylamide gel electrophoresis (SDS-PAGE) analysis. It was confirmed that protein whose size was similar to deduced molecular weight from nucleotide sequence ( 50.8 kDa) was expressed only in the case of clone that harbored pHMC818 with the induction. Cells from 50 ml broth were harvested by the centrifugation (1500 x g, 10 minutes), washed once and suspended in 2 ml of hmc buffer (200 mM Tris-HCl (pH 8.2)). Cells were disrupted by French press homogenizer (Ohtake Works) at 1500 kgf/cm² to yield a crude lysate. After the centrifugation of the crude lysate, a supernatant fraction was recovered and used as a crude extract for an enzymatic analysis. In the only case of induced lysate of pHMC818 clone, a white pellet was spun down and was recovered. Enzyme assay for 3-hydroxy-3-methylglutaryl-CoA (HMG-CoA) synthase was performed by the photometric assay according to the method by Stewart *et al*. (J. Biol. Chem. 241(5), 1212-1221, 1966). In all the crude extract, the activity of 3-hydroxy-3-methylglutaryl-CoA synthase was not detected. As a result of SDS-PAGE analysis of the crude extract, an expressed protein band that had found in expressed broth was disappeared. Subsequently the white pellet that was recovered from the crude lysate of induced pHMC818 clone was solubilized with 8 M guanidine-HCl, and then subjected to SDS-PAGE analysis. The expressed protein was recovered in the white pellet, and this suggested that expressed protein would form an inclusion body.

Next, an expression experiment in more mild condition was conducted. Cells were grown in LB medium at 28 °C and the induction was performed by the addition of 0.1 mM of IPTG. Subsequently, incubation was continued further for 3.5 hours at 28 °C and then the cells were harvested. Preparation of the crude extract was the same as the previous protocol. Results are summarized in TABLE 5. It was shown that HMG-CoA synthase activity was only observed in the induced culture of the recombinant strain harboring *hmc* gene, and this suggested that the cloned *hmc* gene encodes HMG-CoA synthase.

**TABLE 5**

| Enzymatic characterization of *hmc* cDNA clone | | |
|---|---|---|
| plasmid | IPTG | µ mol of HMG-CoA / minute / mg-protein |
| pHMC818 | - | 0 |
| | + | 0.146 |
| pET11c | - | 0 |
| | + | 0 |

### Example 8 Cloning of hmg (3-hydroxymethyl-3-glutaryl-CoA reductase) gene

Cloning protocol of *hmg* gene was almost the same as the *hmc* gene shown in Example 2 to 7. At first, the PCR primers whose sequences were shown in TABLE 6 based on the common sequences of HMG-CoA reductase genes from other species were synthesized.

**TABLE 6**

| Sequence of primers used in the cloning of *hmg* gene |
|---|
| Red1 ; GCNTGYTGYGARAAYGTNATHGGNTAYATGCC (sense primer) (SEQ ID NO: 19) |
| Red2 ; ATCCARTTDATNGCNGCNGGYTTYTTRTCNGT (antisense primer) (SEQ ID NO: 20) |
| (N=A, C, G or T; R=A or G, Y=C or T, H=A, T or C, D=A, G or T) |

After the PCR reaction of 25 cycles of 95 °C for 30 seconds, 54 °C for 30 seconds and 72°C for 30 seconds by using ExTaq (Takara Shuzo) as a DNA polymerase, reaction mixture was applied to agarose gel electrophoresis. PCR band that has a desired length was recovered and purified by QIAquick (QIAGEN) according to the method by the manufacturer and then ligated to pUC57 vector (MBI Fermentas). After the transformation of competent *E. coli* DH5α, 7 white colonies were selected and the plasmids were isolated from those transformants. As a result of sequencing, it was found that all the clones had a sequence whose deduced amino acid sequence was similar to known HMG-CoA reductase genes. One of the isolated cDNA clones was named as pRED1219 and used for further study.

Next, a genomic fragment containing 5'- and 3'- adjacent region of *hmg* gene was cloned with the Genome Walker kit (Clontech). The 2.5 kb fragment of 5' adjacent region (pREDPVu1226) and 4.0 kb fragment of 3' adjacent region of *hmg* gene (pREDEVd1226) were cloned. Based on the sequence of the insert of pREDPVu1226, PCR primers whose sequence were shown in TABLE 7 were synthesized.

**TABLE 7**

| Sequence of primers used in the cloning of cDNA of *hmg* gene |
|---|
| Red8 ; GGCCATTCCACACTTGATGCTCTGC (antisense primer) (SEQ ID NO: 21) |
| Red9 ; GGCCGATATCTTTATGGTCCT (sense primer) (SEQ ID NO: 22) |

Subsequently a cDNA fragment containing a long portion of *hmg* cDNA sequence was cloned by a PCR method by using Red 8 and Red 9 as PCR primers and the cDNA pool prepared in Example 2 and thus cloned plasmid was named as pRED107. PCR condition was as follows; 25 cycles of 30 seconds at 94 °C, 30 seconds at 55 °C and 1 minute at 72 °C.

Southern blot hybridization study was performed to clone genomic sequence which contains the entire *hmg* gene from *P. rhodozyma*. Probe was prepared by labeling a template DNA, pRED107 with DIG multipriming method. Hybridization was performed with the method specified by the manufacturer. As a result, labeled probe hybridized to two bands that had 12 kb and 4 kb in their lengths. As a result of sequencing of pREDPVu1226, *EcoR*I site wasn't found in the cloned *hmg* region. This suggested that another species of *hmg* gene (that has 4 kb of hybridized *EcoR*I fragment) existed on the genome of *P. rhodozyma* as found in other organisms.

Next, a genomic library consisting of 9 to 23 kb of *EcoR*I fragment in the λDASHII vector was constructed. The packaged extract was infected to *E. coli* XL1 Blue, MRA(P2) strain (Stratagene) and over-laid with NZY medium poured onto LB agar medium. About 5000 plaques were screened by using 0.6 kb fragment of *Stu*I- digested pRED107 as a probe. 4 plaques were hybridized to the labeled probe. Then a phage lysate was prepared and DNA was purified with Wizard lambda purification system according to the method specified by the manufacturer (Promega) and was digested with *EcoR*I to isolate 10 kb of *EcoR*I fragment and to clone in *EcoR*I-digested and CIAP-treated pBluescriptII KS-(Stratagene). Eleven white colonies were selected and subjected to a colony PCR by using Red9 and -40 universal primer (Pharmacia). Template DNA for a colony PCR was prepared by heating cell suspension in which picked-up colony was suspended in 10 µl of sterilized water for 5 minutes at 99 °C prior to a PCR reaction (PCR condition; 25 cycles of 30 seconds at 94 °C, 30 seconds at 55 °C and 3 minutes at 72 °C). One colony gave 4 kb of a positive PCR band, and it suggested that this clone had an entire region containing *hmg* gene. A plasmid from this positive clone was prepared and named as pRED611. Subsequently deletion derivatives of pRED611 were made up for sequencing. By combining the sequence obtained from deletion mutants with the sequence obtained by a primer-walking procedure, the nucleotide sequence of 7285 base pairs which contains *hmg* gene from *P. rhodozyma* was determined (SEQ ID NO: 2). The *hmg* gene from *P. rhodozyma* consists of 10 exons and 9 introns. The deduced amino acid sequence of 1092 amino acids in its length (SEQ ID NO: 7) showed an extensive homology to known HMG-CoA reductase (53.0 % identity to HMG-CoA reductase from *Ustilago maydis*).

### Example 9 Expression of carboxyl-terminal domain of hmg gene in E. coli

Some species of prokaryotes have soluble HMG-CoA reductases or related proteins (Lam *et al*., J. Biol. Chem. 267, 5829-5834, 1992). However, in eukaryotes, HMG-CoA reductase is tethered to the endoplasmic reticulum via an amino-terminal membrane domain (Skalnik *et al*., J. Biol. Chem. 263, 6836-6841, 1988). In fungi (i.e. *Saccharomyces cerevisiae* and the smut fungus, *Ustilago maydis*) and in animals, the membrane domain is large and complex, containing seven or eight transmembrane segments (Croxen *et al*. Microbiol. 140, 2363-2370, 1994). In contrast, the membrane domains of plant HMG-CoA reductase proteins have only one or two transmembrane segments (Nelson *et al*. Plant Mol. Biol. 25, 401-412, 1994). Despite the difference in the structure and sequence of the transmembrane domain, the amino acid sequences of the catalytic domain are conserved across eukaryotes, archaebacteria and eubacteria.

Croxen *et al*. showed that C-terminal domain of HMG-CoA reductase derived from the maize fungal pathogen, *Ustilago maydis* was expressed in active form in *E. coli* (Microbiology, 140, 2363-2370, 1994). The inventors of the present inventiontried to express a C-terminal domain of HMG-CoA reductase from *P. rhodozyma* in *E. coli* to confirm its enzymatic activity.

At first, the PCR primers whose sequences were shown in TABLE 8 were synthesized to clone a partial cDNA fragment of *hmg* gene. The sense primer sequence corresponds to the sequence which starts from 597th amino acid (glutamate) residue, and a length of protein and cDNA which was expected to obtain was 496 amino acids and 1.5 kb, respectively.

**TABLE 8**

| Sequence of primers used in the cloning of a partial cDNA of *hmg* gene |
|---|
| Red54 ; GGTACCGAAGAAATTATGAAGAGTGG (sense primer) (SEQ ID NO: 23) |
| Red55 ; CTGCAGTCAGGCATCCACGTTCACAC (antisense primer) (SEQ ID NO: 24) |

The PCR condition was as follows; 25 cycles of 95 °C for 30 seconds, 55 °C for 30 seconds and 72 °C for 3 minutes. As a template, 0.1 µg of cDNA pool obtained in Example 2 and as a DNA polymerase, ExTaq polymerase were used. Amplified 1.5 kb fragment was recovered and cloned in pMOSBlue T-vector (Novagen). Twelve independent clones from white colonies of *E. coli* DH5α transformants were selected and plasmids were prepared from those transformants. As a result of restriction analysis, all the clones were selected for a further selection by sequencing. One clone did not have an amino acid substitution all through the coding sequence and was named as pRED908.

Next, 1.5 kb fragment obtained by *Kpn*I- and *Pst*I- digestion of pRED908 was ligated to pQE30 (QIAGEN) digested by the same pairs of restriction enzymes, and transformed to *E. coli* KB822. As a result of restriction analysis, plasmid that had a correct structure (pRED1002) was recovered. Then, competent *E. coli* M15 (pREP4) cells (QIAGEN) were transformed and one clone that had a correct structure was selected for further study.

For an expression study, strain M15 (pREP4) (pRED1002) and vector control strain M15 (pREP4) (pQE30) were cultivated in 100 ml of LB medium at 30 °C until OD at 600 nm reached to 0.8 (about 5 hours) in the presence of 25 µg/ml of kanamycin and 100 µg/ml of ampicillin. Then, the broth was divided into two portions of the same volume, and then 1 mM of IPTG was added to one portion. Cultivation continued for further 3.5 hours at 30 °C. Twenty five µl of the broth was removed from induced- and uninduced- culture of *hmg* clone and vector control cultures and subjected to SDS-PAGE analysis. It was confirmed that protein whose size was similar to deduced molecular weight from nucleotide sequence (52.4 kDa) was expressed only in the case of clone that harbored pRED1002 with the induction. Cells from 50 ml broth were harvested by the centrifugation (1500 x g, 10 minutes), washed once and suspended in 2 ml of hmg buffer (100 mM potassium phosphate buffer (pH 7.0) containing 1 mM of EDTA and 10 mM of dithiothreitol). Cells were disrupted by French press (Ohtake Works) at 1500 kgf/cm² to yield a crude lysate. After the centrifugation of the crude lysate, a supernatant fraction was recovered and used as a crude extract for enzymatic analysis. In the only case of induced lysate of pRED1002 clone, a white pellet was spun down and was recovered. Enzyme assay for 3-hydroxy-3-methylglutaryl-CoA (HMG-CoA) reductase was performed by the photometric assay according to the method by Servouse *et al*. (Biochem. J. 240, 541-547, 1986). In all the crude extract, the activity of 3-hydroxy-3-methylglutaryl-CoA synthase was not detected. As a result of SDS-PAGE analysis for the crude extract, expressed protein band that had found in expressed broth was disappeared. Next, the white pellet that was recovered from the crude lysate of induced pRED1002 clone was solubilized with an equal volume of 20 % SDS, and then subjected to SDS-PAGE analysis. An expressed protein was recovered in the white pellet, and this suggested that the expressed protein would form an inclusion body.

Next, the expression experiment was performed in more mild condition. Cells were grown in LB medium at 28 °C and the induction was performed by the addition of 0.1 mM of IPTG. Then, incubation was continued further for 3.5 hours at 28 °C and then the cells were harvested. Preparation of the crude extract was the same as the previous protocol. Results are summarized in TABLE 9. It was shown that 30 times higher induction was observed, and this suggested that the cloned *hmg* gene codes HMG-CoA reductase.

**TABLE 9**

| Enzymatic characterization of *hmg* cDNA clone | | |
|---|---|---|
| plasmid | IPTGµ mol of NADPH / minute / mg-protein | |
| pRED1002 | - | 0.002 |
| | + | 0.059 |
| pQE30 | - | 0 |
| | + | 0 |

### Example 10 Cloning of mevalonate kinase (mvk) gene

A cloning protocol of *mvk* gene was almost the same as the *hmc* gene shown in Example 2 to 7. At first, the PCR primers whose sequence were shown in TABLE 10, based on the common sequences of mevalonate kinase genes from other species were synthesized.

**TABLE 10**

| Sequence of primers used in the cloning of *mvk* gene |
|---|
| Mk1 ; GCNCCNGGNAARGTNATHYTNTTYGGNGA (sense primer) (SEQ ID NO: 25) |
| Mk2 ; CCCCANGTNSWNACNGCRTTRTCNACNCC (antisense primer) (SEQ ID NO: 26) |
| (N=A, C, G or T; R=A or G, Y=C or T, H=A, T or C, S=C or G, W=A or T) |

After the PCR reaction of 25 cycles of 95 °C for 30 seconds, 46 °C for 30 seconds and 72°C for 15 seconds by using ExTaq as a DNA polymerase, the reaction mixture was applied to agarose gel electrophoresis. A 0.6 kb of PCR band whose length was expected to contain a partial *mvk* gene was recovered and purified by QIAquick according to the method indicated by the manufacturer and then ligated to pMOSBlue T-vector. After a transformation of competent *E. coli* DH5α cells, 4 white colonies were selected and plasmids were isolated. As a result of sequencing, it was found that one of the clones had a sequence whose deduced amino acid sequence was similar to known mevalonate kinase genes. This cDNA clone was named as pMK128 and used for further study.

Next, a partial genomic clone which contained *mvk* gene was cloned by PCR. The PCR primers whose sequence were shown in TABLE 11, based on the internal sequence of pMK128 were synthesized.

**TABLE 11**

| Sequence of primers used in the cloning of genomic DNA containing *mvk* gene |
|---|
| Mk5 ; ACATGCTGTAGTCCATG (sense primer) (SEQ ID NO: 27) |
| Mk6 ; ACTCGGATTCCATGGA (antisense primer) (SEQ ID NOP: 28) |

PCR condition was 25 cycles of 30 seconds at 94 °C, 30 seconds at 55 °C and 1 minute at 72 °C. The amplified 1.4 kb fragment was cloned into pMOSBlue T-vector. As a result of sequencing, it was confirmed a genomic fragment containing *mvk* gene which had typical intron structures could be obtained and this genomic clone was named as pMK224.

Southern blot hybridization study was performed to clone a genomic fragment which contained an entire *mvk* gene from *P. rhodozyma*. Probe was prepared by labeling a template DNA, pMK224 digested by *Nco*I with DIG multipriming method. Hybridization was performed with the method specified by the manufacturer. As a result, the labeled probe hybridized to a band that had 6.5 kb in its lengths. Next, a genomic library consisting of 5 to 7 kb of *EcoR*I fragment was constructed in the λZAPII vector. The packaged extract was infected to *E. coli* XL1Blue, MRF' strain (Stratagene) and over-laid with NZY medium poured onto LB agar medium. About 5000 plaques were screened by using 0.8 kb fragment of *Nco*I- digested pMK224 as a probe. Seven plaques were hybridized to the labeled probe. Then a phage lysate was prepared according to the method specified by the manufacturer (Stratagene) and *in vivo* excision was performed by using *E. col*i XL1BIue MRF' and SOLR strains. Fourteen white colonies were selected and plasmids were isolated from those selected transformants. Then, isolated plasmids were digested by *Nco*I and subjected to Southern blot hybridization with the same probe as the plaque hybridization. The insert fragments of all the plasmids were hybridized to the probe and this suggested that a genomic fragment containing *mvk* gene could be cloned. A plasmid from one of the positive clones was prepared and was named as pMK701. About 3 kb of sequence was determined by the primer walking procedure and it was revealed that 5' end of the *mvk* gene wasn't included into pMK701.

Next, a PCR primer which had a sequence ;
TTGTTGTCGTAGCAGTGGGTGAGAG (SEQ ID NO: 29) was synthesized to clone the 5'-adjacent genomic region of *mvk* gene with the Genome Walker Kit according to the method specified by manufacturer (Clontech). A specific 1.4 kb PCR band was amplified and cloned into pMOSBlue T-vector. All of the transformants of DH5α selected had expected length of the insert. Subsequent sequencing revealed that 5'-adjacent region of *mvk* gene could be cloned. One of the clone was designated as pMKEVR715 and used for further study. As a result of Southern blot hybridization using genomic DNA prepared in example 3, the labeled pMKEVR715 hybridized to 2.7 kb *EcoR*I band. Then a genomic library in which *EcoR*I fragments from 1.4 to 3.0 kb in lengths were cloned into λZAPII was constructed and screened with 1.0 kb of *EcoR*I fragment from pMKEVR715. Fourteen positive plaques were selected from 5000 plaques and plasmids were prepared from those plaques with *in vivo* excision procedure.

The PCR primers whose sequences were shown in TABLE 12, taken from the internal sequence of pMKEVR715 were synthesized to select a positive clone with a colony PCR.

**TABLE 12**

| PCR primers used for colony PCR to clone 5'-adjacent region of *mvk* gene |
|---|
| Mk17 ; GGAAGAGGAAGAGAAAAG (sense primer) (SEQ ID NO: 30) |
| Mk18 ; TTGCCGAACTCAATGTAG (antisense primer) (SEQ ID NO: 31) |

PCR condition was as follows; 25 cycles of 30 seconds at 94 °C, 30 seconds at 50 °C and 15 seconds at 72 °C. From all the candidates except one clone, the positive 0.5 kb band was yielded. One of the clones was selected and named as pMK723 to determine the sequence of the upstream region of *mvk* gene. After sequencing of the 3'-region of pMK723 and combining with the sequence of pMK701, the genomic sequence of 4.8 kb fragment containing *mvk* gene was determined. The mvk gene consists of 4 introns and 5 exons (SEQ ID NO: 3). The deduced amino acid sequence except 4 amino acids in the amino terminal end (SEQ ID NO: 8) showed an extensive homology to known mevalonate kinase (44.3 % identity to mevalonate kinase from *Rattus norvegicus*).

### Example 11 Expression of mvk gene by the introduction of 1 base at amino terminal region

Although the amino acid sequence showed a significant homology to known mevalonate kinase, an appropriate start codon for *mvk* gene could not be found. This result suggested the cloned gene might be a pseudogene for mevalonate kinase. To confirm this assumption, PCR primers whose sequences are shown in TABLE 13 were synthesized to introduce an artificial nucleotide which resulted in the generation of appropriate start codon at the amino terminal end.

**TABLE 13**

| PCR primers used for the introduction of a nucleotide into *mvk* gene |
|---|
| Mk33 ; GGATCCATGAGAGCCCAAAAAGAAGA (sense primer) (SEQ ID NO: 32) |
| Mk34 ; GTCGACTCAAGCAAAAGACCAACGAC (antisense primer) (SEQ ID NO: 33) |

The artificial amino terminal sequence thus introduced were as follows; NH2-Met-Arg-Ala-Gln. After the PCR reaction of 25 cycles of 95 °C for 30 seconds, 55 °C for 30 seconds and 72 °C for 30 seconds by using ExTaq polymerase as a DNA polymerase. The reaction mixture was subjected to agarose gel electrophoresis. An expected 1.4 kb of PCR band was amplified and cloned into pCR2.1 TOPO vector. After a transformation of competent E. coli TOP10 cells, 6 white colonies were selected and plasmids were isolated. As a result of sequencing, it was found that one clone had only one change of amino acid residue (Asp to Gly change at 81st amino acid residue in SEQ ID NO: 8). This plasmid was named as pMK1130 #3334 and used for further study. Then, the insert fragment of pMK1130 #3334 was cloned into pQE30. This plasmid was named as pMK1209 #3334. After the transformation of expression host, M15 (pREP4), expression study was conducted. M15 (pREP4) (pMK1209 #3334) strain and vector control strain (M15 (pREP4) (pQE30)) were inoculated into 3 ml of LB medium containing 100 µg/ml of ampicillin. After the cultivation at 37 °C for 3.75 hours, cultured broth were divided into two portions. 1 mM IPTG were added to one portion and an incubation was continued for 3 hours. Cells were harvested from 50 µl of broth by the centrifugation and were subjected to SDS-PAGE analysis. Protein which had an expected molecular weight of 48.5 kDa was induced by the addition of IPTG in the culture of M15 (pREP4) (pMK1209 #3334) though no induced protein band was observed in the vector control culture (Fig. 2). This result suggested that activated form of the mevalonate kinase protein could be expressed by the artificial addition of one nucleotide at amino terminal end.

### Example 12 Cloning of the mevalonate pyrophosphate decarboxylase (mpd) gene

A cloning protocol of *mpd* gene was almost the same as the *hmc* gene shown in Example 2 to 7. At first, the PCR primers whose sequence were shown in TABLE 14 based on the common sequences of mevalonate pyrophosphate decarboxylase genes from other species were synthesized.

**TABLE 14**

| Sequence of primers used in the cloning of *mpd* gene |
|---|
| Mpd1 ; HTNAARTAYTTGGGNAARMGNGA (sense primer) (SEQ ID NO: 34) |
| Mpd2 ; GCRTTNGGNCCNGCRTCRAANGTRTANGC (antisense primer) (SEQ ID NO: 35) |
| (N=A, C, G or T; R=A or G, Y=C or T, H=A, T or C, M=A or C) |

After the PCR reaction of 25 cycles of 95 °C for 30 seconds, 50 °C for 30 seconds and 72°C for 15 seconds by using ExTaq as a DNA polymerase, reaction mixture was subjected to agarose gel electrophoresis. A 0.9 kb of PCR band whose length was expected to contain a partial *mpd* gene was recovered and purified by QIAquick according to the method prepared by the manufacturer and then ligated to pMOSBlue T-vector. After a transformation of competent *E. coli* DH5α cells, 6 white colonies were selected and plasmids were isolated. Two of 6 clones had an expected length of insert. As a result of sequencing, it was found that one of the clones had a sequence whose deduced amino acid sequence was similar to known mevalonate pyrophosphate decarboxylase genes. This cDNA clone was designated as pMPD129 and used for further study.

Next, a partial genomic fragment which contained *mpd* gene was cloned by PCR. As a result of PCR whose condition was the same as that of the cloning of a partial cDNA fragment the amplified 1.05 kb fragment was obtained and was cloned into pMOSBlue T-vector. As a result of sequencing, it was confirmed that a genomic fragment containing *mpd* gene which had typical intron structures have been obtained and this genomic clone was named as pMPD220.

Southern blot hybridization study was performed to clone a genomic fragment which contained the entire *mpd* gene from *P. rhodozyma*. Probe was prepared by labeling a template DNA, pMPD220 digested by *Kpn*I, with DIG multipriming method. Hybridization was performed with the method specified by the manufacturer. As a result, the probe hybridized to a band that had 7.5 kb in its lengths. Next, a genomic library consisting of from 6.5 to 9.0 kb of *EcoR*I fragment in the λZAPII vector was constructed. The packaged extract was infected to *E. coli* XL1Blue, MRF' strain and over-laid with NZY medium poured onto LB agar medium. About 6000 plaques were screened by using the 0.6 kb fragment of *Kpn*I- digested pMPD220 as a probe. 4 plaques were hybridized to the labeled probe. Then a phage lysate was prepared according to the method specified by the manufacturer (Stratagene) and *in vivo* excision was performed by using *E. coli* XL1Blue MRF' and SOLR strains. Each 3 white colonies derived from 4 positive plaques were selected and plasmids were isolated from those selected transformants. Then, isolated plasmids were subjected to a colony PCR method whose protocol was the same as that in example 8. PCR primers whose sequences were shown in TABLE 14, depending on the sequence found in pMPD129 were synthesized and used for a colony PCR.

**TABLE 15**

| Sequence of primers used in the colony PCR to clone a genomic *mpd* clone |
|---|
| Mpd7 ; CCGAACTCTCGCTCATCGCC (sense primer) (SEQ ID NO: 36) |
| Mpd8 ; CAGATCAGCGCGTGGAGTGA (antisense primer) (SEQ ID NO: 37) |

PCR condition was almost the same as the cloning of mvk gene; 25 cycles of 30 seconds at 94 °C, 30 seconds at 50 °C and 10 seconds at 72 °C. All the clone except one produced a positive 0.2kb PCR band. A plasmid was prepared from one of the positive clones and the plasmid was named as pMPD701 and about 3 kb of sequence thereof was determined by the primer walking procedure (SEQ ID NO: 4). There existed an ORF consisted of 402 amino acids (SEQ ID NO: 9) whose sequence was similar to the sequences of known mevalonate pyrophosphate decarboxylase (52.3 % identity to mevalonate pyrophosphate decarboxylase from *Schizosaccaromyces pombe*). Also determined was a 0.4 kb of 5'-adjacent region which was expected to include its promoter sequence.

### Example 13 Cloning of farnesyl pyrophosphate synthase (fps) gene

A cloning protocol of *fps* gene was almost the same as the *hmc* gene shown in Example 2 to 7. At first, the PCR primers whose sequence were shown in TABLE 16 based on the common sequences of farnesyl pyrophosphate synthase genes from other species were synthesized.

**TABLE 16**

| Sequence of primers used in the cloning of *fps* gene |
|---|
| Fps1 ; CARGCNTAYTTYYTNGTNGCNGAYGA (sense primer) (SEQ ID NO: 38) |
| Fps2 ; CAYTTRTTRTCYTGDATRTCNGTNCCDATYTT (antisense primer) (SEQ ID NO: 39) |
| (N=A, C, G or T; R=A or G, Y=C or T, D=A, G or T) |

After the PCR reaction of 25 cycles of 95 °C for 30 seconds, 54 °C for 30 seconds and 72°C for 30 seconds by using ExTaq as a DNA polymerase, a reaction mixture was applied to agarose gel electrophoresis. A PCR band that has a desired length (0.5 kb) was recovered and purified by QIAquick according to the method prepared by the manufacturer and then ligated to pUC57 vector. After a transformation of competent *E. coli* DH5α cells, 6 white colonies were selected and plasmids were then isolated. One of the plasmids which had desired length of an insert fragment was sequenced. As a result, it was found that this clone had a sequence whose deduced amino acid sequence was similar to known farnesyl pyrophosphate synthase genes. This cDNA clone was named as pFPS107 and used for further study.

Next, a genomic fragment was cloned by PCR by using the same primer set of Fps1 and Fps2. The same PCR condition as the case of cloning of a partial cDNA was used. A 1.0 kb band yielded was cloned and sequenced. This clone contained the same sequence with the pFPS107 and some typical intron fragments. This plasmid was named as pFPS113 and used for a further experiment.

Then, also cloned was a 5'- and 3'- adjacent region containing *fps* gene with the method described in Example 8. At first, the PCR primers whose sequences were shown in TABLE 17 were synthsized.

**TABLE 17**

| Sequences of primers used for a cloning of adjacent region of *fps* gene |
|---|
| Fps7 ; ATCCTCATCCCGATGGGTGAATACT (sense for downstream cloning) (SEQ ID NO: 40) |
| Fps9 ; AGGAGCGGTCAACAGATCGATGAGC (antisense for upstream cloning) (SEQ ID NO: 41) |

Amplified PCR bands were isolated and cloned into pMOSBlue T-vector. As a result of sequencing, it was found that the 5'-adjacent region that had 2.5 kb in its length and 3'-adjacent region that had 2.0 kb in its length were cloned. These plasmids were named as pFPSSTu117 and pFPSSTd117, respectively. After sequencing of both plasmids, it was found that an ORF that consisted of 1068 basepairs with 8 introns. Deduced amino acid sequence showed an extensive homology to the known farnesyl pyrophosphate synthase from other species. Based on the sequence determined, two PCR primers were synthesized with the sequences shown in TABLE 17 to clone a genomic *fps* clone and cDNA clone for *fps* gene expression in *E. coli*.

**TABLE 18**

| Sequences of primers used for a cDNA and genomic *fps* cloning |
|---|
| Fps27 ; GAATTCATATGTCCACTACGCCTGA (sense primer) (SEQ ID NO: 42) |
| Fps28 ; GTCGACGGTACCTATCACTCCCGCC (antisense primer) (SEQ ID NO: 43) |

PCR condition was as follows; 25 cycles of 30 seconds at 94 °C, 30 seconds at 50 °C and 30 seconds at 72 °C. One cDNA clone that had a correct sequence was selected as a result of sequencing analysis of clones obtained by PCR and was named as pFPS113. Next, Southern blot hybridization study was performed to clone a genomic fragment which contained the entire *fps* gene from *P. rhodozyma*. Probe was prepared by labeling a template DNA, pFPS113 with DIG multipriming method. As a result, labeled probe hybridized to a band that had about 10 kb in its length.

Next, a genomic library consisting of 9 to 15 kb of *EcoR*I fragment was constructed in a λDASHII vector. The packaged extract was infected to *E. coli* XL1 Blue, MRA(P2) strain (Stratagene) and over-laid with NZY medium poured onto LB agar medium. About 10000 plaques were screened by using the 0.6 kb fragment of *Sac*I- digested pFPS113 as a probe. Eight plaques were hybridized to the labeled probe. Then a phage lysate was prepared according to the method specified by the manufacturer (Promega). All the plaques were subjected to a plaque PCR using Fps27 and Fps28 primers. Template DNA for a plaque PCR was prepared by heating 2 µl of solution of phage particles for 5 minutes at 99 °C prior to a PCR reaction. PCR condition is the same as that of pFPS113 cloning hereinbefore. All the plaques gave a 2 kb of positive PCR band, and this suggested that these clones had an entire region containing *fps* gene. One of the λDNA that harbored *fps* gene was digested with *EcoR*I to isolate 10 kb of *EcoR*I fragment and to clone in *EcoR*I-digested and CIAP-treated pBluescriptII KS- (Stratagene). Twelve white colonies from transformed *E. coli* DH5α cells were selected and plasmids were prepared from these clones and subjected to colony PCR by using the same primer sets of Fps27 and Fps28 and the same PCR condition. Two kb of positive band were yielded from 3 of 12 candidates. One clone was cloned and named as pFPS603. It was confirmed that sequence of *fps* gene which was previously determined from the sequence of pFPSSTu117 and pFPSStd117 were almost correct although they had some PCR errors. Finally, it was determined the nucleotide sequence of 4092 base pairs which contains *fps* gene from *P. rhodozyma* (Fig. 3), and an ORF which consisted of 365 amino acids with 8 introns was found (SEQ ID NO: 5). Deduced amino acid sequence (SEQ ID NO: 10) showed an extensive homology to known FPP synthase (65 % identity to FPP synthase from *Kluyveromyces lactis*).

## Claims

1. An isolated DNA sequence, which codes for an enzyme involved in the mevalonate pathway or the pathway from isopentenyl pyrophosphate to farnesyl pyrophosphate.

2. An isolated DNA sequence according to claim 1, wherein said enzyme has an activity selected from the group consisting of 3-hydroxy-3-methylglutaryl-CoA synthase activity, 3-hydroxy-3-methylglutaryyl-CoA reductase activity, mevalonate kinase activity, mevalonate pyrophosphate decarboxylase activity and farnesyl pyrophosphate synthase activity.

3. An isolated DNA sequence according to claim 1 or 2, which is characterized in that
(a) the said DNA sequence codes for the said enzyme having an amino acid sequence selected from the group consisting of those described in SEQ ID NOs: 6, 7, 8, 9 and 10, or
(b) the said DNA sequence codes for a variant of the said enzyme selected from (i) an allelic variant, and (ii) an enzyme having one or more amino acid addition, insertion, deletion and/or substitution and having the stated enzyme activity.

4. An isolated DNA sequence according to any one of claims 1-3, which can be derived from a gene of *Phaffia rhodozyma* and is selected from:
(i) a DNA sequence represented in SEQ ID NOs: 1, 2, 4 or 5;
(ii) an isocoding or an allelic variant for the DNA sequence represented in SEQ ID NOs: 1, 2, 4 or 5; and
(iii) a derivative of a DNA sequence represented in SEQ ID NOs: 1, 2, 4 or 5 with addition, insertion, deletion and/or substitution of one or more nucleotide(s), and coding for a polypeptide having the said enzyme activity.

5. An isolated DNA sequence, which is selected from:
(i) a DNA sequence represented in SEQ ID NO: 3;
(ii) an isocoding or an allelic variant for the DNA sequence represented in SEQ ID NO: 3; and
(iii) a derivative of a DNA sequence represented in SEQ ID NO: 3 with addition, insertion, deletion and/or substitution of one or more nucleotide(s), and coding for a polypeptide having the mevalonate kinase activity.

6. An isolated DNA sequence as claimed in claim 1 or 2 and which is selected from:
(i) a DNA sequence which hybridizes under standard conditions with a sequence as shown in SEQ ID Nos: 1 - 10 or its complementary strand or fragments thereof; and
(ii) a DNA sequence which do not hybridize as defined in (i) because of the degeneration of the genetic code but which codes for polypeptives having exactly the same amino acid sequence as shown in SEQ ID Nos: 1 - 10 or those encoded by a DNA sequence as defined above under (i).

7. A vector or plasmid comprising a DNA sequence as defined in any of claims 1-6.

8. A host cell which has been transformed or transfected by a DNA sequence as claimed in anyone of claims 1 to 6, or a vector or plasmid as claimed in claim 7.

9. A process for producing an enzyme involed in the mevalonate pathway or the pathway from isopentenyl pyrophosphate to farnesyl pyrophosphate, which comprises culturing a host cell as claimed in claim 8, under the conditions conductive to the production of said enzyme.

10. A process for the production of isoprenoids or carotenoids, preferably astaxanthin, which comprises cultivating a host cell as claimed in claim 8 under suitable culture conditions.


SEQUENCE LISTING

(1) GENERAL INFORMATION:

(i) APPLICANT: F.HOFFMANN-LA ROCHE. AG

(ii) TITLE OF INVENTION: Improvement of microbiological
carotenoid production and biological materials therefor

(iii) NUMBER OF SEQUENCES: 43

(iv) CORRESPONDENCE ADDRESS:
(A) ADDRESSEE:
(B) STREET: Grezacherstrasse 124
(C) CITY: BASLE
(E) COUNTRY: SWITZERLAND
(F) ZIP: CH-4002

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(ix) TELECOMMUNICATION INFORMATION:
(A) TELEPHONE: 061-688 25 11
(B) TELEFAX: 061-688 13 95
(C) TELEX: 962292/965542 hlr c

(2) INFORMATION FOR SEQ ID NO:1:

(i) SEQUENCE CHARACTERISTICS:
(A) LENGTH: 6370 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Phaffia rhodozyma
(B) STRAIN: ATCC96594

(ix) FEATURE:
(A) NAME/KEY: exon
(B) LOCATION: 1441..1466

(ix) FEATURE:
(A) NAME/KEY: intron
(B) LOCATION: 1467..1722

(ix) FEATURE:
(A) NAME/KEY: exon
(B) LOCATION: 1723..1813

(ix) FEATURE:
(A) NAME/KEY: intron
(B) LOCATION: 1814..1914

(ix) FEATURE:
(A) NAME/KEY: exon
(B) LOCATION: 1915..2535

(ix) FEATURE:
(A) NAME/KEY: intron
(B) LOCATION: 2536..2621

(ix) FEATURE:
(A) NAME/KEY: exon
(B) LOCATION: 2622..2867

(ix) FEATURE:
(A) NAME/KEY: intron
(B) LOCATION: 2868..2942

(ix) FEATURE:
(A) NAME/KEY: exon
(B) LOCATION: 2943..3897

(ix) FEATURE:
(A) NAME/KEY: intron
(B) LOCATION: 3898..4030

(ix) FEATURE:
(A) NAME/KEY: exon
(B) LOCATION: 4031..4516

(ix) FEATURE:
(A) NAME/KEY: intron
(B) LOCATION: 4517..4616

(ix) FEATURE:
(A) NAME/KEY: exon
(B) LOCATION: 4617..4909

(ix) FEATURE:
(A) NAME/KEY: intron
(B) LOCATION: 4910..5007

(ix) FEATURE:
(A) NAME/KEY: exon
(B) LOCATION: 5008..5081

(ix) FEATURE:
(A) NAME/KEY: intron
(B) LOCATION: 5082..5195

(ix) FEATURE:
(A) NAME/KEY: exon
(B) LOCATION: 5196..5446

(ix) FEATURE:
(A) NAME/KEY: intron
(B) LOCATION: 5447..5523

(ix) FEATURE:
(A) NAME/KEY: exon
(B) LOCATION: 5524..5756

(ix) FEATURE:
(A) NAME/KEY: polyA_site

(B) LOCATION: 6173

(xi) SEQUENCE DESCRIPTION: SEQ ID NO:1:

GGAAGACATG ATGGTGTGGG TGTGAGTATG AGCGTGAGCG TGGGTATGGG CCTGGGTGTG 60

GGTATGAGCG GTGGTGGTGA TGGATGGATG GGTGGGTGGC GTGGAGGGGT CCGTGCGGCA 120

AGATGTTTTC TCTGGGTAGG AGCGTTCTGC ATTGGGGCAG GAGAAAAAAT AGTGTGGTTA 180

CGGGAGATCG TGGTTACATC AAGCCATCGT CACTGTAAGG CTCTGTAAGG CTCGGTTGTT 240

AAGAAGGTAA CCAAGTGTAA TCACTTGGTT CGCGGGGTGA CACTTAGGCT CTGGCGATTA 300

ATATATCTGA AGCAGACCAA ACTATTAACA ATATACTTTT GGATAAGAGG TTTCAACAAG 360

AATCTCAGCT TGAGGAAAAC TCTTATCCAA GAAGGCGCGA GGGCGTCCCC GTTTTATATC 420

AGGACCCCTC GCGCATTTGG TCTGCCACTA AAGATATACA TATGACGAGC CTAGAGAGGC 480

TCGAGATCAC GAAAACTAAA AAGATGAAGC ATGAACCATG CAAACTAGAG CATGATGGAA 540

AATGGGCGAA GAGGCATAAG GGATGGAGGG AACGAATAGC CTGTAGGGGT AACCCACGTA 600

AGAGAACACG TGATACTTAA CCCGTATCCC TGACAGTCAC GGTGTTTCTT GAGAGTCAGT 660

AATGTCCAGC TGTGACCTCA CGTGACTAAA CCCGACACGT GTGCTTCGAC CGAGGTGGGA 720

CGATCTTTTT TTTGGGGGGA GAAACCGAGT GGGACGATAG AGAGGACTAC GGAGAACTGT 780

AGTGAATTGT AGTGCGCTCA CTACGGAGAG TTCTAGTTGA GCAAGCGATG TGATTTTCAA 840

TACAATCCCG GACTACAAGC TCTCTAATAG AGCTCTATAA TAGAAGGACA AAAGTCGTCC 900

CACTCCTATC TCCCGCGCGT TTTAATAGAG ACCGATTGTT TTTTTCCCTA ATGTTTTATT 960

TTCTTTCCCC GATCGGCTCA TTTTTCTTCT CTCCGCGTAT TCTTCACACA ACGCTCCCTC 1020

CGATCTTTTT TCTTCTTGTT CCTGTTCCTC TTCGTCTCCT TCCATTGTCT TCTTTCCTTC 1080

CTTCCTTCCT TCTTGCCTCT AGCCAGCTTC AACAGCGACG TCTCTCTCTC TCTGTGTGGT 1140

GATCTCCGAC TGTAGTGTCT CTCTCGGTCA CTTTCACGAA TCAACTTCGT TTCTTTTCTG 1200

ATCGATCGGT CGTCTTTCCC TCAATCCGTG CATACACTCA CACTTACACT CACACCCACA 1260

CACTCAAACA CGCTAAATAA TCAGATCCGT CTCCCCTTCT TGATCTCCTT CGGCTTAGGC 1320

AATGGCTTCC TTGTTCGGCC TCCGGCGGTC CTCAAACGAG CAGCCGCGCT CTCCTCTGCT 1380

CATCCAATCG AAGTCATCCT TTCTACCTTT GTCGTGGTCA CCTTGACGTA CTTTCAGTTG 1440

ATGTACACCA TCAAGCACAG TAATTTGTAC GTCCGATCAT CTATTTGTCG TGTTCTCCTT 1500

AGTCTCTTTC TCTTCCTCCT TTGTCTTTCG CGTCAGCGTG GCTGGATTTC CGTCTCCATG 1560

TCATTTCCCT TATTTCCTCT TCCTGTCATT TGTTCCTCTA CTTTTCTTTC TCTACCTCCT 1620

TTCCCTGTCG TTTGCTTTCC TTCGCCAGTT GACCACCGAT CCTCAGGATT CATGGCTAAC 1680

ATGCCCAACA CAAACTTGCA TATCATCTCT CTTCGTCCAC AGTCTTTCTC AGACGATTAG 1740

CACACAATCT ACCACCAGCT GGGTCGTCGA TGCGTTCTTC TCTTTGGGAT CCAGATACCT 1800

TGACCTCGCG AAGGTTAGTC AGTTGACCCT CTCATGCTTC TTTTCTCTCA GTCTTGTGTG 1860

TGCGCATATA CCCACTCATA GACATCTTCG TACGCTGCAC TTTCCCTCCC TTAGCAAGCA 1920

GACTCGGCCG ATATCTTTAT GGTCCTCCTC GGTTACGTCC TTATGCACGG CACATTCGTC 1980

CGACTGTTCC TCAACTTTCG TCGGATGGGC GCAAACTTTT GGCTGCCAGG CATGGTTCTT 2040

GTCTCGTCCT CCTTTGCCTT CCTCACCGCC CTCCTCGCCG CCTCGATCCT CAACGTTCCG 2100

ATCGACCCGA TCTGTCTCTC GGAAGCACTT CCCTTCCTCG TGCTCACCGT CGGATTTGAC 2160

AAGGACTTTA CCCTCGCAAA ATCTGTGTTC AGCTCCCCAG AAATCGCACC CGTCATGCTT 2220

AGACGAAAGC CGGTGATCCA ACCAGGAGAT GACGACGATC TCGAACAGGA CGAGCACAGC 2280

AGAGTGGCCG CCAACAAGGT TGACATTCAG TGGGCCCCTC CGGTCGCCGC CTCCCGTATC 2340

GTCATTGGCT CGGTCGAGAA GATCGGGTCC TCGATCGTCA GAGACTTTGC CCTCGAGGTC 2400

GCCGTCCTCC TTCTCGGAGC CGCCAGCGGG CTCGGCGGAC TCAAGGAGTT TTGTAAGCTC 2460

GCCGCGTTAA TTTTGGTGGC CGACTGCTGC TTCACCTTTA CCTTCTATGT CGCCATCCTC 2520

ACCGTCATGG TCGAGGTAAG CCTTTTCTTC AAGTTTCTTG CTGTCATTTT CCTTTCGACA 2580

CGTATGCTCA TCTTTCGTTT CCGTCTCTCT CACCTTTCCA GGTTCACCGA ATCAAGATCA 2640

TCCGGGGCTT CCGACCGGCC CACAATAACC GAACACCGAA TACTGTGCCC TCTACCCCTA 2700

CTATCGACGG TCAATCTACC AACAGATCCG GCATCTCGTC AGGGCCTCCG GCCCGACCGA 2760

CCGTGCCCGT GTGGAAGAAA GTCTGGAGGA AGCTCATGGG CCCAGAGATC GATTGGGCGT 2820

CCGAAGCTGA GGCTCGAAAC CCGGTTCCAA AGTTGAAGTT GCTCTTAGTA AGTAAACTTC 2880

CTTTGTTCTT CTCATCATTC TTTATCTCCG AATCCTGACG TCGGACCCTT CTCGATTCAA 2940

AGATCTTGGC CTTTCTTATC CTTCATATCC TCAACCTTTG CACGCCTCTG ACCGAGACCA 3000

CAGCTATCAA GCGATCGTCT AGCATACACC AGCCCATTTA TGCCGACCCT GCTCATCCGA 3060

TCGCACAGAC AAACACGACG CTCCATCGGG CGCACAGCCT AGTCATCTTT GATCAGTTCC 3120

TTAGTGACTG GACGACCATC GTCGGAGATC CAATCATGAG CAAGTGGATC ATCATCACCC 3180

TGGGCGTGTC CATCCTGCTG AACGGGTTCC TCCTAAAAGG GATCGCTTCT GGCTCTGCTC 3240

TCGGACCCGG TCGTGCCGGA GGAGGAGGAG CTGCCGCCGC CGCCGCCGTC TTGCTCGGAG 3300

CGTGGGAAAT CGTCGATTGG AACAATGAGA CAGAGACCTC AACGAACACT CCGGCTGGTC 3360

CACCCGGCCA CAAGAACCAG AATGTCAACC TCCGACTCAG TCTCGAGCGG GATACTGGTC 3420

TCCTCCGTTA CCAGCGTGAG CAGGCCTACC AGGCCCAGTC TCAGATCCTC GCTCCTATTT 3480

CACCGGTCTC TGTCGCGCCC GTCGTCTCCA ACGGTAACGG TAACGCATCG AAATCGATTG 3540

AGAAACCAAT GCCTCGTTTG GTGGTCCCTA ACGGACCAAG ATCCTTGCCT GAATCACCAC 3600

CTTCGACGAC AGAATCAACC CCGGTCAACA AGGTTATCAT CGGTGGACCG TCCGACAGGC 3660

CTGCCCTAGA CGGACTCGCC AATGGAAACG GTGCCGTCCC CCTTGACAAA CAAACTGTGC 3720

TTGGCATGAG GTCGATCGAA GAATGCGAAG AAATTATGAA GAGTGGTCTC GGGCCTTACT 3780

CACTCAACGA CGAAGAATTG ATTTTGTTGA CTCAAAAGGG AAAGATTCCG CCGTACTCGC 3840

TGGAAAAAGC ATTGCAGAAC TGTGAGCGGG CGGTCAAGAT TCGAAGGGCG GTTATCTGTA 3900
GGTCTTTTTC TCCTTTGAAT TTCAAGCCTT GGAGGAGAGG AAAGTGCTTC GGGGTACAAT 3960
ACAGGTTGTG CAAACAAACC AAGAGAAACT AAAGAAAACT TTCTTCTCCT CTCTCTCCCC 4020
TCGACGTCAG CCCGAGCATC CGTTACTAAG ACGCTGGAAA CCTCGGACTT GCCCATGAAG 4080
GATTACGACT ACTCGAAAGT GATGGGCGCA TGCTGTGAGA ACGTTGTCGG ATATATGCCT 4140
CTCCCTGTCG GAATCGCTGG TCCACTTAAC ATTGATGGCG AGGTCGTCCC CATCCCGATG 4200
GCCACCACCG AGGGAACTCT CGTGGCCTCG ACGTCGAGAG GTTGCAAAGC GCTCAACGCG 4260
GGTGGCGGAG TGACCACCGT CATCACCCAG GATGCGATGA CGAGAGGACC GGTGGTGGAT 4320
TTCCCTTCGG TCTCTCAGGC CGCACAGGCC AAACGATGGT TGGATTCGGT CGAAGGAATG 4380
GAGGTTATGG CCGCTTCGTT CAACTCGACT TCTAGATTCG CCAGGTTGCA GAGCATCAAG 4440
TGTGGAATGG CCGGCCGATC GCTATACATC CGTTTGGCGA CCAGTACCGG AGATGCGATG 4500
GGAATGAACA TGGCTGGTGA GTGCGACGAG TTTTCTTTGT TCTTCTTGTG CGGACCATGT 4560
TTTCTCATCC AGCCAATTCA TTCTTCATTC CTTCTCGGTG TTTGGCAACC TTTTAGGTAA 4620
AGGAACGGAG AAAGCTTTGG AAACCCTGTC CGAGTACTTC CCATCCATGC AGATCCTTGC 4680
TCTTTCTGGT AACTACTGTA TCGACAAGAA GCCTTCTGCC ATCAACTGGA TTGAGGGCCG 4740
TGGAAAGTCC GTGGTGGCCG AGTCGGTGAT CCCTGGAGCG ATCGTCAAGT CTGTCCTCAA 4800
GACAACGGTT GCGGATCTCG TCAACTTGAA CATTAAGAAA AACTTGATCG GAAGTGCCAT 4860
GGCAGGCAGC ATTGGAGGAT TCAACGCCCA CGCGTCGAAT ATTTTGACTG TGCGTACTTC 4920
TCTTTCCATA TTCGTCCTCG TTTAATTTCT TTTCTGTCCA GTCTTATGAC GTCTGATTGG 4980
TTCTTCTTTT CACCCACACA CATACAGTCA ATCTTCTTGG CTACAGGTCA GGATCCTGCA 5040
CAGAATGTGG AGTCCTCAAT GTGCATGACA TTGATGGAGG CGTACGTTTT TTGTTTTGTT 5100
TTCCTTCTTT TTCCATATGT TTCTACTTCT ACTTTCTTCC CGAGTCCGCC AAGCTGATAC 5160
CTTTATACGG TCCTTCTCTT TCTCATGACG AGTAGTGTGA ACGACGGAAA AGATCTACTC 5220
ATCACCTGCT CGATGCCGGC GATCGAGTGC GGAACGGTCG GTGGAGGAAC TTTCCTCCCT 5280
CCGCAAAACG CCTGTTTGCA GATGCTCGGT GTCGCAGGTG CCCATCCAGA TTCGCCCGGT 5340
CACAATGCTC GTCGACTAGC AAGAATCATC GCTGCCAGTG TGATGGCTGG AGAGTTGAGT 5400
TTGATGAGTG CTTTGGCCGC TGGTCATTTA ATCAAGGCCC ACATGAGTAA GTCTGCCACC 5460
TTTTGATAAT CAAAAGGGTC GTGGTACTGG TGTCACTGAC TGGTGACTCT TCCTGTCATG 5520
CAGAGCACAA TCGATCGACA CCTTCGACTC CTCTACCGGT CTCACCGTTG GCGACCCGAC 5580
CGAACACGCC GTCCCACCGG TCGATTGGAT TGCTCACACC GATGACGTCT TCCGCATCGG 5640
TCGCCTCGAT GTTCTCTGGG TTCGGTAGTC CGTCGACGAG CTCGCTCAAG ACGGTAGGTA 5700
GCATGGCTTG CGTCAGGGAA CGAGGGGACG AGACGAGTGT GAACGTGGAT GCCTGAACTG 5760
GGGACTCCCT TTTCTTGGTA TCCCTTCCGT TTTTCTTTCG GCCTTTGAAT CCTGTATTCT 5820

TGTCCGTTTT TTCATCTTCT CTTCCTGGTT CTCCTTCTCT CGTTCATCTG CAAAAACAAA 5880

ATTCAATCGC ATCGGTCTCT GGCATTCCAT TTGGGTTTCA AAATCAAATC AATCTCTATC 5940

TACTATCTCA AATATCTTTT TTTCATCTTT TGATTCATTT CTGTTGAAAA CTGTCTTGCC 6000

CTTCTCCTAC TTCTTATCTC TGCCTTCTTG CCAAAGTTCA ATTCGTTGTC CATCTGTGCA 6060

CTCTGATCTA TCAGTCTGTA TCAAGTACGC TCTTAAATCT GTAATTGGCT CTCGGAGGTG 6120

TCTCGTCATC TCACATATGG CTGGCGATAT GATGTGTCGG TTTCTTCCCC TCCAACAAAG 6180

GCGACGTGGC TCCTTCATCA ATCTTTGGCG CAAGCTCTCA AAATTCTCCA AAACGGCTGA 6240

CTAAGCAAGG TTTCCAAGTA CTCTCAAACC GAGCAAGGCC ATCCATCCTC AAATCAACTT 6300

GTGAAACCCT TTGTGGATAG ACCGTCCAAA CCGAGCTCTT CCCAATCTTC GCCTCCCCTT 6360

CTTCCTGCAG 6370

(2) INFORMATION FOR SEQ ID NO:2:

(i) SEQUENCE CHARACTERISTICS:
(A) LENGTH: 4775 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Phaffia rhodozyma
(B) STRAIN: ATCC96594

(ix) FEATURE:
(A) NAME/KEY: exon
(B) LOCATION: 1305..1361

(ix) FEATURE:
(A) NAME/KEY: intron
(B) LOCATION: 1362..1504

(ix) FEATURE:
(A) NAME/KEY: exon
(B) LOCATION: 1505..1522

(ix) FEATURE:
(A) NAME/KEY: intron
(B) LOCATION: 1523..1699

(ix) FEATURE:
(A) NAME/KEY: exon
(B) LOCATION: 1700..1826

(ix) FEATURE:
(A) NAME/KEY: intron
(B) LOCATION: 1827..1920

(ix) FEATURE:
(A) NAME/KEY: exon
(B) LOCATION: 1921..2277

(ix) FEATURE:
(A) NAME/KEY: intron
(B) LOCATION: 2278..2351

(ix) FEATURE:
(A) NAME/KEY: exon
(B) LOCATION: 2352..2409

(ix) FEATURE:
(A) NAME/KEY: intron
(B) LOCATION: 2410..2497

(ix) FEATURE:
(A) NAME/KEY: exon
(B) LOCATION: 2498..2504

(ix) FEATURE:
(A) NAME/KEY: intron
(B) LOCATION: 2505..2586

(ix) FEATURE:
(A) NAME/KEY: exon
(B) LOCATION: 2587..2768

(ix) FEATURE:
(A) NAME/KEY: intron
(B) LOCATION: 2769..2851

(ix) FEATURE:
(A) NAME/KEY: exon
(B) LOCATION: 2852..2891

(ix) FEATURE:
(A) NAME/KEY: intron
(B) LOCATION: 2892..2985

(ix) FEATURE:
(A) NAME/KEY: exon
(B) LOCATION: 2986..3240

(ix) FEATURE:
(A) NAME/KEY: intron
(B) LOCATION: 3241..3325

(ix) FEATURE:
(A) NAME/KEY: exon
(B) LOCATION: 3326..3493

(ix) FEATURE:
(A) NAME/KEY: intron
(B) LOCATION: 3494..3601

(ix) FEATURE:
(A) NAME/KEY: exon
(B) LOCATION: 3602..3768

(ix) FEATURE:
(A) NAME/KEY: polyA_site
(B) LOCATION: 4043

(xi) SEQUENCE DESCRIPTION: SEQ ID NO:2:

CATCGAAGAG AGCGAAGTGA TTAGGGAAGC CGAAGAGGCA CTAACAACGT GGTTGTATAT 60

GTGTGTTTAT GAGTGTTATA TCGTCAAGAA CGAAGTCCAT TCATTTAGCT AGACAGGGAG 120

AGAGGGAGAA ACGTACGGGT TTACCCTATT GGACCAGTCT AAAGAGAGAA CGAGAGTTTT 180
TGGGTCGGTC ACCTGAAGAG TTTGAACCTC CACAAGTTTA TTCTAGATTA TTTCCGGGGG 240
TATGTGAAGG ATAATGTCAA ACTTTGTCCA GATTGAAGAA GGCAAGAAAG GAAAGGGGCG 300
AACGAGAGTA TCGTCCCATC TATGGGTGAC CAGTCGACCT TCTGCATCGG CGATCCCGAG 360
AATGGAAGGT TCCGATGGAT CAGAAGTAGG TTTCCTAAGC TCAAACATAG GTCATTGCGA 420
GTGAGATACA TATGCAGACT GATATGCTAG TCAAACCGAA CGAGATTTCT CTGTTTGCTT 480
TCAAAAAGAC GAACCAACCA TTTCATGTCC AAGATGGCAG GTCCTTCGAT TCTTTGAAGC 540
TCCTCCCTGA TGCGGACAGA AAAGAATAAA AAGTAGACAG ACTGTCAAGT CGACAGCGCA 600
AGTTTATCAA GCTGAGCGAG AAAACTCGAA CTTACATACC TTGGCCGTCA GTTCTGTAGA 660
CCAAGCATCG GCCTTTCCTC TTTGCGGCAG GTGTACGCGT TGGCTCACCA TCGTCACTCT 720
CGTCTCCTGA CCCGTTGCTT TCCTTGACAG CAGTCTGTTC CACAGGTTTC TCTAACTGAT 780
AGGTCCCAAC AGCAAAGATA TCTGGATGTC TATGTGAGAA CTCTACTGAG TCGGCAGAGT 840
ACACCGTATC GATATAGGCG AGTGAGGAAG CTTTGAAAGG TGAAGAAGTA GCGAAAGATC 900
ATCAGCGAAT GAGGACTATG ACAAAAAAGA AATTTTCGTA TAATCCACTG GACAAATCAC 960
CTTCCATCGT GTCCTCCAAG AGGGTTTCGT CTGAAACGTA AGGACGAGGT ATTGATAGAT 1020
GATTGACCTT GAGTACGCGG ATGGACAAGG AACGAGCCCA CTCCCAGGGC TATGTAACAC 1080
CACACGTGAC TCCACTTGAA TTGCGGCAGA TAAACGAAGT CTTACGATCG GACGACTTTG 1140
TAACCATTTA GTTATTTACC CGTCTTGTTT TCTTACTTTG ATCGTCCCAT TTTAGACACA 1200
AAAAAAGAAG CCAGAAGAGA AAAGAATAAA ACGTCTACCG TGTTCTCTCC GAATTCTTAC 1260
CACACCCACA AAACCATACA CAATCTCAAT CTAGATATCC AGTTATGTAC ACTTCTACTA 1320
CCGAACAGCG ACCCAAAGAT GTTGGAATTC TCGGTATGGA GGTATGTTGT TCAATTCTGT 1380
TTGTGTTCAA TCTTTAATCA TCTTTAGTCG ACTGACCGGT TCTTCCTTTT TTTTTCTTCA 1440
TCAAACAAAA CAACCCTTCT CGATTCATGT CATCTTTCTT TCCAATGCGC TACTCCTTCT 1500
GTAGATCTAC TTTCCTCGAC GAGTGCGTAA CTATTCTCTC TTCTGCATTC TCTCTCTATT 1560
CCCATGTTCG ATCCCTCGCC CTCATATGGG CGACTGTTTC ATCTCTTTTG CTTCCGTCCA 1620
TTCTTCTTTG ATCTTGTTCA TTTTCTACTA ATATCTCCCG ACGCGAAATA CAACACTGAC 1680
CGCGATTTCT CTCGATCAGG CCATCGCTCA CAAGGATCTC GAGGCTTTTG ATGGGGTTCC 1740
TTCCGGAAAG TACACCATCG GTCTCGGCAA CAACTTCATG GCCTTCACCG ACGACACTGA 1800
GGACATCAAC TCGTTCGCCT TGAACGGTCA GTCTCTTCCG TTTCAGCAAT CGACAGGAAA 1860
AAGGCCCAAG CGCATCTCAC TGACACCTTT CTCCGTTTTG CAATTCCATT TGATTGTTAG 1920
CTGTTTCCGG TCTTCTATCA AAGTACAACG TTGATCCCAA GTCAATCGGT CGAATTGATG 1980
TCGGAACTGA GTCCATCATT GACAAGTCCA AATCTGTCAA GACAGTCCTT ATGGACTTGT 2040
TCGAGTCCCA CGGCAACACA GATATTGAGG GTATCGACTC CAAGAATGCC TGCTACGGTT 2100

CTACCGCGGC CCTGTTCAAT GCCGTCAACT GGATCGAGTC ATCCTCTTGG GACGGAAGAA 2160
ATGCCATTGT CTTCTGCGGA GACATTGCCA TCTACGCCGA GGGTGCTGCC CGACCTGCCG 2220
GAGGTGCTGG TGCTTGCGCC ATCCTCATCG GACCCGACGC TCCCGTCGTC TTCGAGCGTG 2280
AGTTCCAATC CGTCATTTTC TTCCACGGCA GCGGCTGAAA CAACCCTTAT CCGTCATTCT 2340
CATCAATCTA GCCGTCCACG GAAACTTCAT GACCAACGCT TGGGACTTCT ACAAGCCTAA 2400
TCTTTCTTCG TATGTTCAAA TTTTGAAGTT TGCGCTTGGG AGAGTCTTAC ACTAATTCGG 2460
GGTGCTCGTA TCCTTCGAAT CGTTTGTTGC TTTATAGTGA ATACGTTCGT CTGCGCACCT 2520
CCTATATTTA GTTTTTGATC AAATATTGTC CATTGAATTA ACTCTGAAAC CTTCTCCTCC 2580
AAATAGCCCA TTGTCGATGG ACCTCTCTCC GTCACTTCCT ACGTCAACGC CATTGACAAG 2640
GCCTATGAAG CTTACCGAAC AAAGTATGCC AAGCGATTTG GAGGACCCAA GACTAACGGT 2700
GTCACCAACG GACACACCGA GGTTGCCGGT GTCAGTGCTG CGTCGTTCGA TTACCTTTTG 2760
TTCCACAGGT AAGCGTCATC TTCTGTATTC TCCTTAAATT CAACCGATCA ACGGAGTTAA 2820
TTCGTGTCAT CATATTATCT TGTTGGAACA GTCCTTACGG AAAGCAGGTT GTCAAAGGCC 2880
ACGGCCGACT TGTAAGCAGT CTTTTTGTAA CTCTTAGCTT GCAGATAAAA ACTTTTAGGT 2940
TTCTGGTACT CATTATTTAT GCATCTCTTG AATCACCTTA TCTAGTTGTA CAATGACTTC 3000
CGAAACAACC CCAACGACCC GGTTTTTGCT GAGGTGCCAG CCGAGCTTGC TACTTTGGAC 3060
ATGAAGAAAA GTCTTTCAGA CAAGAATGTC GAGAAATCTC TGATTGCTGC CTCCAAGTCT 3120
TCTTTCAACA AGCAGGTTGA GCCTGGAATG ACCACCGTCC GACAGCTCGG AAACTTGTAC 3180
ACCGCCTCTC TCTTCGGTGC TCTCGCAAGT TTGTTCTCTA ATGTTCCTGG TGACGAGCTC 3240
GTAAGTCTTG ATCTCTATCC CAATCATCTC TTCCTTATCA ATTGAACTGA ACTCTTTTCT 3300
TTAATGCTGG CTTTCTCTTG AACAGGTCGG CAAGCGCATT GCTCTCTACG CCTACGGATC 3360
TGGAGCTGCT GCTTCTTTCT ATGCTCTTAA GGTCAAGAGC TCAACCGCTT TCATCTCTGA 3420
GAAGCTTGAT CTCAACAACC GATTGAGCAA CATGAAGATT GTCCCCTGTG ATGACTTTGT 3480
CAAAGCTCTG AAGGTACGTT GGATAATGAC TTTTTTTGTG GACCGTGGTC TTTGTCAACC 3540
GCTAACAACC TTCTTGAATC GGTCTCTTTT GGTTTGAAAT TCGCTCGGCG CTTCGACACA 3600
GGTCCGAGAA GAGACTCACA ACGCCGTGTC ATATTCGCCC ATCGGTTCGC TTGACGATCT 3660
CTGGCCTGGA TCGTACTACT TGGGAGAGAT TGACAGCATG TGGCGTCGAC AGTACAAGCA 3720
GGTCCCTTCT GCTTGAACGG GATATTAAAA GTTTCAAAAG TTATGAAAGA GGTCGGCGAA 3780
GATTCAAAAT AAATAAATAT AACACCTTGC TTTTTGGCTT GTTTTCCTTC TTCACTCTCG 3840
TTTCCGATGT GTTTCCTCCG TTTCTTCCCT CTTTTGTTCC TTTTTCCTCC CTCTTTTGGT 3900
TACAATCTCT TTGGGTTTTA CAGGCTGGCA ATCTCTGTAC AATCTTCGTT CGCGTGATCC 3960
GACATAGATA CCGTTGTGGC ATACACCTTG CGTCTTACAT CTTTTGAGAG CTTCGGAGGT 4020
GATCTTGATG AAGAAAATTC ACCATTGACT CCCATCTCTT GAATGTCCTG ACTAAATTGA 4080

ATTGGAAGCA ACTTATATGA AGAGCAAATT GATGGATCCA GAAAGGAACA AGTCTAGAAA 4140

TCAGTGATTT GTGCGAAAAA TCAGCAAATG CCGCGCTGAG CCGCTCGCTG GGGAGTAGAC 4200

ATTGCCCATG CGCGTGATGT TGTCTGACCG TTCTCCTCCA TTCCCCCACT CTCAACCTTC 4260

CTCTCTTTGA GAATCGAAGA AGAAGGCGAA GAAAACCTGA CTTGATCCTT TACAGGGTGT 4320

TTCTTTTGTT CGTATCTGAG TTACTTTTCC TCCTTTCCTT CCTGCTTGAG TGAATGACTG 4380

ATCTGACTCC TCCGCCTACC TCGGCGACTG GGCTATATCT TGAGGATAGA ATATCCCCCT 4440

GACAATCCCA TTTCTCAAGA TTCTTTCAAA CAAGAAAACT AGTTCCAATC AATAGATCAT 4500

CTGATCAACC TTGTGTGAAC ATAATCATCT GCAGAAGCAC TGAACTGAGA AAGTCTTCCT 4560

CAGAGGAAAG AGAATACTAG ATAAGATCAT TCGGTTGGGA AGGTAAAGGA ATGAAGTCTG 4620

GTTCTGGGTT TAGCTCTGGT TCCGTAGGGG GTTCGACTAT AGTTTCTTCT GTTCGACTAG 4680

AAACAGGAGA AACCGTACAT GTAAATGGTA TGATATTCTT GTCTCTGTAT CATGTCCCGC 4740

TCATCTCTTT GTTTGCAAGT CACTCTGGAG AATTC 4775

(2) INFORMATION FOR SEQ ID NO:3:

(i) SEQUENCE CHARACTERISTICS:
(A) LENGTH: 4135 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Phaffia rhodozyma
(B) STRAIN: ATCC96594

(ix) FEATURE:
(A) NAME/KEY: exon
(B) LOCATION: 1021..1124

(ix) FEATURE:
(A) NAME/KEY: intron
(B) LOCATION: 1125..1630

(ix) FEATURE:
(A) NAME/KEY: exon
(B) LOCATION: 1631..1956

(ix) FEATURE:
(A) NAME/KEY: intron
(B) LOCATION: 1957..2051

(ix) FEATURE:
(A) NAME/KEY: exon
(B) LOCATION: 2052..2366

(ix) FEATURE:
(A) NAME/KEY: intron

(B) LOCATION: 2367..2446

(ix) FEATURE:
(A) NAME/KEY: exon
(B) LOCATION: 2447..2651

(ix) FEATURE:
(A) NAME/KEY: intron
(B) LOCATION: 2652..2732

(ix) FEATURE:
(A) NAME/KEY: exon
(B) LOCATION: 2733..3188

(ix) FEATURE:
(A) NAME/KEY: polyA_site
(B) LOCATION: 3284

(xi) SEQUENCE DESCRIPTION: SEQ ID NO:3:

ACTGACTCGG CTACCGGAAA ATATCTTTTC AGGACGCCTT GATCGTTTTG GACAACACCA 60

TGATGTCACC ATATCTTCAG CGGCCGTTGG AGCTAGGAGT AGACATTGTA TACGACTCTG 120

GAACAAAGTA TTTGAGTGGA CACCACGATC TCATGGCTGG TGTGATTACT ACTCGTACTG 180

AGGAGATTGG GAAGGTTCGT GCTTGCTTGC TTTGAATGTC GTGCCTAAAG CCATTGCCAT 240

AAGACAGAGT CTGATCTATG TCGTTTGCCT ACAACAGAGA ATGGCCTGGT TCCCAAATGC 300

TATGGGAAAT GCATTGTCTC CGTTCGACTC GTTCCTTCTT CTCCGAGGAC TCAAAACACT 360

TCCTCTCCGA CTGGACAAGC AGCAGGCCTC ATCTCACCTG ATCGCCTCGT ACTTACACAC 420

CCTCGGCTTT CTTGTTCACT ACCCCGGTCT GCCTTCTGAC CCTGGGTACG AACTTCATAA 480

CTCTCAGGCG AGTGGTGCAG GTGCCGTCAT GAGCTTTGAG ACCGGAGATA TCGCGTTGAG 540

TGAGGCCATC GTGGGCGGAA CCCGAGTTTG GGGAATCAGT GTCAGTTTCG GAGCCGTGAA 600

CAGTTTGATC AGCATGCCTT GTCTAATGAG GTTAGTTCTT ATGCCTTCTT TTCGCGCCTT 660

CTAAAATTTC TGGCTGACTA ATTGGGTCGG TCTTTCCGTT CTTGCATTTC AGTCACGCAT 720

CTATTCCTGC TCACCTTCGA GCCGAGCGAG GTCTCCCCGA ACATCTGATT CGACTGTGTG 780

TCGGTATTGA GGACCCTCAC GATTTGCTTG ATGATTTGGA GGCCTCTCTT GTGAACGCTG 840

GCGCAATCCG ATCAGTCTCT ACCTCAGATT CATCCCGACC GCTCACTCCT CCTGCCTCTG 900

ATTCTGCCTC GGACATTCAC TCCAACTGGG CCGTCGACCG AGCCAGACAG TTCGAGCGTG 960

TTAGGCCTTC TAACTCGACA GCCGGCGTCG AAGGACAGCT TGCCGAACTC AATGTAGACG 1020

ATGCAGCCAG ACTTGCGGGC GATGAGAGCC AAAAAGAAGA AATTCTTGTC AGTGCACCGG 1080

GAAAGGTCAT TCTGTTCGGC GAACATGCTG TAGGCCATGG TGTTGTGAGT GAGAAATGAA 1140

AGCTTTATGC TCTCATTGCA TCTTAACTTT TCCTCGCCTT TTTTGTTCTC TTCATCCCGT 1200

CTTGATTGTA GGGATGCCCC CCTTTGCCCC TTTCCCCTTC TTGCATCTGT CTATATTTCC 1260

TTATACATTT CGCTCTTAAG AGCGTCTAGT TGTACCTTAT AACAACCTTT GGTTTTAGCA 1320

TCCTTTGATT ATTCATTTCT CTCATCCTTC GGTCAGAGGC TTTCGGCCAT CTTTACGTCT 1380

GATTAGATTG TAATAGCAAG AACTATCTTG CTAAGCCTTT TCTCTTCCTC TTCCTCCTAT 1440
ATAAATCGAA TTCACTTTCG GACATGTTTA TTTTGGGGAA ATCATCAAGG GGTGGGGGGC 1500
CAATCCCGAC ACTAATTTTC TGCTCACGTC AAAACTCAGC GTTCAGAATC AGTCACTGAC 1560
CCTGATACGT GTCTCTATGT GTGTGGGTGT ACGTGCGAAT TGTGACTCGA CGTTCTACGC 1620
TTAAAAACAG ACCGGGATCG CTGCTTCCGT TGATCTTCGA TGCTACGCTC TTCTCTCACC 1680
CACTGCTACG ACAACAACAT CATCGTCGTT ATCGTCTACA AACATTACCA TCTCCCTAAC 1740
GGACCTGAAC TTTACGCAGT CTTGGCCTGT TGATTCTCTT CCTTGGTCAC TTGCGCCTGA 1800
CTGGACTGAG GCGTCTATTC CAGAATCTCT CTGCCCGACA TTGCTCGCCG AAATCGAAAG 1860
GATCGCTGGT CAAGGTGGAA ACGGAGGAGA AAGGGAGAAG GTGGCAACCA TGGCATTCTT 1920
GTATTTGTTG GTGCTATTGA GCAAAGGGAA GCCAAGGTAG GTTTTTTCTG TCTCTTCTTT 1980
TTGCCTATAA AGACTCTTAA CTGACGGAGA AAGTGTTGGG TTTCTTCCTT CGGGGGTTCA 2040
ATCAATTAAA GTGAGCCGTT CGAGTTGACG GCTCGATCTG CGCTTCCGAT GGGAGCTGGT 2100
CTGGGTTCAT CCGCCGCTCT ATCGACCTCT CTTGCCCTAG TCTTTCTTCT CCACTTTTCT 2160
CACCTCAGTC CAACGACGAC TGGCAGAGAA TCAACAATCC CGACGGCCGA CACAGAAGTA 2220
ATTGACAAAT GGGCGTTCTT AGCTGAAAAA GTCATCCATG GAAATCCGAG TGGGATTGAT 2280
AACGCGGTCA GTACGAGAGG AGGCGCTGTT GCTTTCAAAA GAAAGATTGA GGGAAAACAG 2340
GAAGGTGGAA TGGAAGCGAT CAAGAGGTAC GCAGACACGG TGCTTCATAT GCCATACTCC 2400
AGTCTGATTG ACCCATGATG AACGTCTTTC TACATTTCGA ATATAGCTTC ACATCCATTC 2460
GATTCCTCAT CACAGATTCT CGTATCGGAA GGGATACAAG ATCTCTCGTT GCAGGAGTGA 2520
ATGCTCGACT GATTCAGGAG CCAGAGGTGA TCGTCCCTTT GTTGGAAGCG ATTCAGCAGA 2580
TTGCCGATGA GGCTATTCGA TGCTTGAAAG ATTCAGAGAT GGAACGTGCT GTCATGATCG 2640
ATCGACTTCA AGTTAGTTCT TGTTCCTTTC AAGACTCTTT GTGACATTGT GTCTTATCCA 2700
TTTCATCTTC TTTTTTCTTC CTTCTTCTGC AGAACTTGGT CTCCGAGAAC CACGCACACC 2760
TAGCAGCACT TGGCGTGTCC CACCCATCCC TCGAAGAGAT TATCCGGATC GGTGCTGATA 2820
AGCCTTTCGA GCTTCGAACA AAGTTGACAG GCGCCGGTGG AGGTGGTTGC GCTGTAACCC 2880
TGGTGCCCGA TGGTAAAGTC TCTCCTTTTC TCTTCCGTCC AAGCGACACA TCTGACCGAT 2940
GCGCATCCTG TACTTTTGGT CAACCAGACT TCTCGACTGA AACCCTTCAA GCTCTTATGG 3000
AGACGCTCGT TCAATCATCG TTCGCCCCTT ATATTGCCCG AGTGGGTGGT TCAGGCGTCG 3060
GATTCCTTTC ATCAACTAAG GCCGATCCGG AAGATGGGGA GAACAGACTT AAAGATGGGC 3120
TGGTGGGAAC GGAGATTGAT GAGCTAGACA GATGGGCTTT GAAAACGGGT CGTTGGTCTT 3180
TTGCTTGAAC GAAAGATAGG AAACGGTGAT TAGGGTACAG ATCCTTTGCT GTCATTTTTA 3240
CAAAACACTT TCTTATGTCT TCATGACTCA ACGTATGCCC TCATCTCTAT CCATAGACAG 3300
CACGGTACCT CTCAGGTTTC AATACGTAAG CGTTCATCGA CAAAACATGC GGCACACGAA 3360

AACGAGTGGA TATAAGGGAG AAGAGAGATA TTAGAGCGAA AAAGAGAAGA GTGAGAGAGG 3420

AAAAAAATAA CCGAGAACAA CTTATTCCGG TTTGTTAGAA TCGAAGATCG AGAAATATGA 3480

AGTACATAGT ATAAAGTAAA GAAGAGAGGT TTACCTCAGA GGTGTGTACG AAGGTGAGGA 3540

CAGGTAAGAG GAATAATTGA CTATCGAAAA AAGAGAACTC AACAGAAGCA CTGGGATAAA 3600

GCCTAGAATG TAAGTCTCAT CGGTCCGCGA TGAAAGAGAA ATTGAAGGAA GAAAAAGCCC 3660

CCAGTAAACA ATCCAACCAA CCTCTTGGAC GATTGCGAAA CACACACACG CACGCGGACA 3720

TATTTCGTAC ACAAGGACGG GACATTCTTT TTTTATATCC GGGTGGGGAG AGAGAGGGTT 3780

ATAGAGGATG AATAGCAAGG TTGATGTTTT GTAAAAGGTT GCAGAAAAAG GAAAGTGAGA 3840

GTAGGAACAT GCATTAAAAA CCTGCCCAAA GCGATTTATA TCGTTCTTCT GTTTTCACTT 3900

CTTTCCGGGC GCTTTCTTAG ACCGCGGTGG TGAAGGGTTA CTCCTGCCAA CTAGAAGAAG 3960

CAACATGAGT CAAGGATTAG ATCATCACGT GTCTCATTTG ACGGGTTGAA AGATATATTT 4020

AGATACTAAC TGCTTCCCAC GCCGACTGAA AAGATGAATT GAATCATGTC GAGTGGCAAC 4080

GAACGAAAGA ACAAATAGTA AGAATGAATT ACTAGAAAAG ACAGAATGAC TAGAA 4135

(2) INFORMATION FOR SEQ ID NO:4:

(i) SEQUENCE CHARACTERISTICS:
(A) LENGTH: 2767 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Phaffia rhodozyma
(B) STRAIN: ATCC96594

(ix) FEATURE:
(A) NAME/KEY: exon
(B) LOCATION: 401..451

(ix) FEATURE:
(A) NAME/KEY: intron
(B) LOCATION: 452..633

(ix) FEATURE:
(A) NAME/KEY: exon
(B) LOCATION: 634..876

(ix) FEATURE:
(A) NAME/KEY: intron
(B) LOCATION: 877..1004

(ix) FEATURE:
(A) NAME/KEY: exon
(B) LOCATION: 1005..1916

(ix) FEATURE:

(A) NAME/KEY: polyA_site
(B) LOCATION: 2217

(xi) SEQUENCE DESCRIPTION: SEQ ID NO:4:

GAATTCTTCC CGACTGGGCT GATCGACTTG ACTGGAAGAT CTAAGGCGGA GGGATGAAGG 60

AAGTAATTGG AGGGAATGAG GAAAAAAAAA GGCGAGGGAA CGCGGTCTTC TTTCCTGGCA 120

AGGCAATGTC GTGTATCTCT CTTGATTCTT TCGTTGTATC GACGGACCAC ACTCTTTTCG 180

AATGAATATC ACTATCGCAT CCAATGATCG CTATACATGG CATTTACATA TGCCAGACAT 240

CGCTGAGAAA GAGAGAACAT TCCTTTGGAA AAAGCCTACT GTGCCTGAAG TCAGGCTGAT 300

GTTGATTAAA CGTCTTTCCC CATCCTAAGC AGACAAACAA CTTCTTTTCG TTCAACACAC 360

CACCTCTCTC CGAAAAAGCT CTTCAATCCA GTCCATTAAG ATGGTTCATA TCGCTACTGC 420

CTCGGCTCCC GTTAACATTG CGTGTATCAA GGTCCGTCTG CATTGTGAAT GCTGCTCGTT 480

TGCCTTGTGT GCGTTTGGTG GATCTGAAAG AACCCTTGCT TGAACCATTC CATCTCTGCT 540

CTTTTTCTTC CTGTCCTTTC CTTTTTCTCA CGACAAAAAA ACCACCTGGA CCCTTTGTGT 600

TCCTTTCCAT TGGTGTTCAT ACACCTAACA CAGTACTGGG GTAAACGGGA TACCAAGTTG 660

ATTCTCCCTA CAAACTCCTC CTTGTCTGTC ACTCTCGACC AGGATCACCT CCGATCGACG 720

ACGTCTTCTG CTTGTGACGC CTCGTTCGAG AAGGATCGAC TTTGGCTTAA CGGGATCGAG 780

GAGGAGGTCA AGGCTGGTGG TCGGTTGGAT GTCTGCATCA AGGAGATGAA GAAGCTTCGA 840

GCGCAAGAGG AAGAGAAGGA TGCCGGTCTG GAGAAAGTGA GTTTTTCTCC TGTGTGCGTG 900

TGTACTCTGT ATAGGTACCG TTGACAGGAC AGTCTTTCTG AAGAGTTTGG ATCTTACTCT 960

TTTTTGGGGG GGTGGTGGTG TTTGAAATAA TGACCAAAAT AAAGCTCTCA TCTTTCAACG 1020

TGCACCTTGC GTCTTACAAC AACTTCCCGA CTGCCGCTGG ACTTGCTTCC TCCGCTTCCG 1080

GTCTAGCTGC GTTGGTCGCC TCGCTCGCCT CGCTCTACAA CCTCCCAACG AACGCATCCG 1140

AACTCTCGCT CATCGCCCGA CAAGGTTCTG GTTCTGCCTG CCGATCGCTC TTCGGCGGGT 1200

TCGTTGCTTG GGAACAGGGC AAGCTTTCCT CTGGAACCGA CTCGTTCGCT GTTCAGGTCG 1260

AGCCCAGGGA ACACTGGCCC TCACTCCACG CGCTGATCTG TGTAGTTTCC GACGAGAAAA 1320

AGACGACGGC CTCGACGGCA GGCATGCAAA CCACGGTGAA CACCTCGCCT TTGCTCCAAC 1380

ACCGAATCGA ACACGTCGTT CCAGCCCGGA TGGAGGCCAT CACCCAGGCG ATCCGGGCCA 1440

AGGATTTCGA CTCGTTCGCA AAGATCACCA TGAAGGACTC CAACCAGTTC CACGCCGTCT 1500

GCCTCGATTC GGAACCCCCG ATCTTTTACT TGAACGATGT CTCCCGATCG ATCATCCATC 1560

TCGTCACCGA GCTCAACAGA GTGTCCGTCC AGGCCGGCGG TCCCGTCCTT GCCGCCTACA 1620

CGTTCGACGC CGGGCCGAAC GCGGTGATCT ACGCCGAGGA ATCGTCCATG CCGGAGATCA 1680

TCAGGTTAAT CGAGCGGTAC TTCCCGTTGG GAACGGCTTT CGAGAACCCG TTCGGGGTTA 1740

ACACCGAAGG CGGTGATGCC CTGAGGGAAG GCTTTAACCA GAACGTCGCC CCGGTGTTCA 1800

GGAAGGGAAG CGTCGCCCGG TTGATTCACA CCCGGATCGG TGATGGACCC AGGACGTATG 1860

GCGAGGAGGA GAGCCTGATC GGCGAAGACG GTCTGCCAAA GGTCGTCAAG GCTTAGACTA 1920

TAGGTTGTTT CTTCTAAATT TGAGCCTTCC TCCCGCCTCC CTTCCACAAG CATAAAACAA 1980

AGGATAAACA AATGAATTAT CAAAATAACT ATAGGTTGTT TCTTCTAAAT TTGAGCCTTC 2040

CTCCCGCCTC CCTTCCACAA GCATAAAACA AAGGATAAAC AAATGAATTA TCAAAATAAA 2100

ATAAAAAGTC TGCCTTCTTT GTTTTGGAAT ACATCTTCTT TGGGACATGA CCCTTCTCCT 2160

TCTTTTCCGT ATACATCTTT TTGGGTATTT CATGGTGATC AAACAACATT GTGATCGAAA 2220

GCAGAGACGG CCATGGTGCT GGCTTTGAGC GTCTGGCGTT TTGTGTGTCC TGCACTTGAG 2280

CAACCCCAAG CTGACCGCTA GGAAAACTCA TTGATGTGAT TTATATCGTA CGATGAAAGA 2340

GAATAAAATG ATAGAAGAAC AAAGAAGAAC AAAGTAGAAG AACGTCTGAG AAGAAAGACA 2400

GGAAAATGAC ACGTACATAG TGTTCGATGA TGAATGATAT AATATTAAAT ATAAAATGAG 2460

GTAAACGTAT AGCATCACGG GATGAACGGA TGAACATGTA GTGGACAAGG TTGGGAAATA 2520

GGAATGTAGA ATCCAAGAAT CGTTGACTGA TGGACGGACG TATGTAAACA GGTACACCCC 2580

AAAGAAAAGA AAGAAAGAAA GAAAGAAAAC ACAAAGCCAA GGAAGTAAAG CAGATGGTCT 2640

TCTAAGAATA CGGCTTCAAA AAGACAGTGA ACACTCGTCG TCGAGGAATG ACAAGAAAAG 2700

TGAGAGACTA CGAAAGGAAG AAACCAAGAC GAAAAGAAGA ACGGAGATCG AACGGACAGA 2760

AATAAAG 2767

(2) INFORMATION FOR SEQ ID NO:5:

(i) SEQUENCE CHARACTERISTICS:
(A) LENGTH: 4092 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Phaffia rhodozyma
(B) STRAIN: ATCC96594

(ix) FEATURE:
(A) NAME/KEY: exon
(B) LOCATION: 852..986

(ix) FEATURE:
(A) NAME/KEY: intron
(B) LOCATION: 987..1173

(ix) FEATURE:
(A) NAME/KEY: exon
(B) LOCATION: 1174..1317

(ix) FEATURE:
(A) NAME/KEY: intron

(B) LOCATION: 1318..1468

(ix) FEATURE:
(A) NAME/KEY: exon
(B) LOCATION: 1469..1549

(ix) FEATURE:
(A) NAME/KEY: intron
(B) LOCATION: 1550..1671

(ix) FEATURE:
(A) NAME/KEY: exon
(B) LOCATION: 1672..1794

(ix) FEATURE:
(A) NAME/KEY: intron
(B) LOCATION: 1795..1890

(ix) FEATURE:
(A) NAME/KEY: exon
(B) LOCATION: 1891..1979

(ix) FEATURE:
(A) NAME/KEY: intron
(B) LOCATION: 1980..2092

(ix) FEATURE:
(A) NAME/KEY: exon
(B) LOCATION: 2093..2165

(ix) FEATURE:
(A) NAME/KEY: intron
(B) LOCATION: 2166..2250

(ix) FEATURE:
(A) NAME/KEY: exon
(B) LOCATION: 2251..2391

(ix) FEATURE:
(A) NAME/KEY: intron
(B) LOCATION: 2392..2488

(ix) FEATURE:
(A) NAME/KEY: exon
(B) LOCATION: 2489..2652

(ix) FEATURE:
(A) NAME/KEY: intron
(B) LOCATION: 2653..2784

(ix) FEATURE:
(A) NAME/KEY: exon
(B) LOCATION: 2785..2902

(ix) FEATURE:
(A) NAME/KEY: polyA_site
(B) LOCATION: 3024

(xi) SEQUENCE DESCRIPTION: SEQ ID NO:5:

CGCCCGGTAT CTTGCCACAG ATGCCGCCGG AGTGTCTGGC GGAGTGCTAG GAACAACGTC 60

ATCTCCATCT GACGAGCAAG CGTACCACAA GCTAGCTCTT CGTCTGTCAG AAGGACATCC 120

ACGCACCTTC CTGGCCTTCG GGGATGGCAC CTTCTCGTCG ACTTCCCATG GCCGTGCCCC 180

TGGCCTTGTG AAGATACTGT TTGCCAAGCT GAGCGCCTCC CCGCTGCTCC AGGTCCGCAA 240

GGTCCGAGAG TATTGGACGT CGAAGATATG TTCAAAGTGT CAGGCGAGTT CTCGGGAGAA 300

AAAAAAAGCG TGGGCTCTGA AACAGTGTGG AAATGTCTAC AAAGTGAGCT GGATTTATTG 360

TGTGTGTATG TGTGTGTGTG TGTATGTTCT GTGTTGGTTG CTCACTGTAC TCTATGCTCT 420

CTCTTAGATT TGGGGAACAG TGCTGTGAAC GCGTCGCGAA ACATGCTGCA CCTAGCCCTT 480

CACCAGAAGG AGAACCAGAG GGCGGGAATG CTGGTGTCTG ACGCTGCTAC TGCTGCTACG 540

CTAGCCGCTG AGGCTGAGGC TGGCAGAAAC TAAATCCATG ACCCATCAGA TCTTGGTGAT 600

TCGTGGTCTG AGGACACCCA AGTCCAAAAG GGCTATATAT CGACCATCAT CCGTTGCGGT 660

CACTCAGTAG TAACTAAAGC TATACATAGG AATGTTCTGA ACTTGATAAC CCTAACACTA 720

CGAAAATATC TCGGAAAATA GATTAATTTC CTTCTCATCT CAAACAAAAG ACACAACACC 780

ATCAATCACG CTCCTTTCAC ACACTCTCCT TTTTGCTCTC TCGTTCGACA GAAAATAACA 840

TCAATAGCCA AATGTCCACT ACGCCTGAAG AGAAGAAAGC AGCTCGAGCA AAGTTCGAGG 900

CTGTCTTCCC GGTCATTGCC GATGAGATTC TCGATTATAT GAAGGGTGAA GGCATGCCTG 960

CCGAGGCTTT GGAATGGATG AACAAGGTTC GTCAAGGGTT TCTTCTTTAT TCTTCTGGTC 1020

TTTGTTTCGG TCGAACTGGC TTTCGAACTT GGCCTTGACC GGTTGGATCT CGGTTGTTGC 1080

GCCAAAACGA TGTCGAAGCA AAACTTACTC TTACCTGTTC GGTTTCCTTC CTTCCGACCT 1140

TCTCTCTACC CTTGCCTCCG ATCGGTCTTA TAGAACTTGT ACTACAACAC TCCCGGAGGA 1200

AAACTCAACC GAGGACTTTC CGTGGTGGAT ACTTATATCC TTCTCTCGCC TTCTGGAAAA 1260

GACATCTCGG AAGAAGAGTA CTTGAAGGCC GCTATCCTCG GTTGGTGTAT CGAGCTTGTA 1320

CGCGTTTTCT TCATTCACCT TTCTTTCTCG TCTTCTACTC TCTTCTCTCG AACTATCTTC 1380

CCTGCGTGTC ATCCTACACG AATCTTTATA CTTACATGTT GGAACATATG CCCTGTTCTT 1440

AATTCACCTC TTTTGTCTCG GATGGTAGCT CCAAGCTTAC TTCTTGGTGG CTGATGATAT 1500

GATGGACGCC TCAATCACCC GACGAGGCCA ACCCTGTTGG TACAAAGTTG TTAGTCCCTT 1560

CTTCTCTTTC TGTCCTCTTT CTTCTGAGCT ATGCCAATTC TTGATTGAAA TCGGTGGTGC 1620

CGTCCGGACT AATCCGTTTG TCGTTTTTAT CATATCTTCT TGCACAAACA GGAGGGAGTG 1680

TCTAACATTG CCATCAACGA CGCGTTCATG CTCGAGGGAG CTATCTACTT TTTGCTCAAG 1740

AAGCACTTCC GAAAGCAGAG CTACTATGTC GATCTGCTAG AGCTCTTCCA CGATGTTTGT 1800

CTCTATTTCT TTTCTTCCTC CCCTCAATAA ACTGTATTTG TGACCATTCT GGATCCTTTC 1860

CTGACGATGA ATCATTCTTC GGATGAGTAG GTTACTTTCC AAACCGAGTT GGGACAGCTC 1920

ATCGATCTGT TGACCGCTCC TGAGGATCAC GTCGATCTCG ACAAGTTCTC CCTTAACAAG 1980

TATGCCCGTC ATATATTCGT TTTGTTGCAT TCACGTCTGA TTGTCAGCTC CGATTATTGA 2040

CTCTGATGGT GATGGTATTG ACCACATCAT GCGATGTTTG ACTTTCTCGT AGGCACCACC 2100

TCATCGTTGT TTACAAGACC GCTTTCTATT CATTCTACCT TCCTGTCGCA CTCGCTATGC 2160

GAATGGTGGG TCTCTCTCTT CAACTGTTCT TCCTGATTTT CTTGACCATC TGTAACATAA 2220
ATCCTTGGAA TTTTGAACTC TATGTCATAG GTCGGCGTGA CAGATGAGGA GGCGTACAAG 2280
CTTGCGCTCT CGATCCTCAT CCCGATGGGT GAATACTTTC AAGTTCAGGA TGATGTGCTC 2340
GACGCGTTCG CTCCTCCGGA GATCCTTGGA AAGATCGGAA CCGACATCTT GGTGCGTTTT 2400
CGTTCCTTCC TTCTACGTTC TGTTTTCTAT CTTCTGACTC CCCGTCCATC ATTTATGCTT 2460
CTGTTAAAAC GTATTGAAAC ATCAAAAGGA CAACAAATGT TCATGGCCTA TCAACCTTGC 2520
ACTCTCTCTC GCCTCGCCCG CTCAGCGAGA GATTCTCGAT ACTTCGTACG GTCAGAAGAA 2580
CTCGGAGGCA GAGGCCAGAG TCAAGGCTCT GTACGCTGAG CTTGATATCC AGGGAAAGTT 2640
CAACGCTTAT GAGTATGTCA TCTTTTTTAA ATTTTCTAAT TTTCTTTTCA TCTCTTGTTC 2700
CCAAGAATTA TTTTGTGAAA GTTCTGGGAC TGAACATGGT GCATCCCTTT GGGTTCACTC 2760
CGCATATGTC TCCCGTTTGA ATAGGCAACA GAGTTACGAG TCGCTGAACA AGTTGATTGA 2820
CAGTATTGAC GAAGAGAAGA GTGGACTCAA GAAAGAAGTC TTCCACAGCT TCCTGGGTAA 2880
GGTCTATAAG CGAAGCAAGT AATTCTCCTC TTTATATGCA AAGGGAAGAT TTTGGCGGGA 2940
GTGATAGGTA GGAAGAGAAG GGAGGGTCAT ATTCATTAGG CATTTCTCTT GCAGATATAG 3000
ATGATCAAAA AGGGATATCG GTCCTCTTCT TTGTTCCGAA TACATAATAA GTCATACGAA 3060
GCCGAACATG ACAAAAGTGG TTCATGAGAT CAAACTTTTT GCATGATCTT CTGCGATTTT 3120
GTACAATTCT CTCGCATCCT ATTAGGATCG AACCAGGAGA AGATGAGAGA AGGAAACCCT 3180
CACCCCGTCA GATAACAAAC GAGAAGTCTC ATCACACACA CACACAGATG AAAGAGAAAA 3240
ATAAACTGAC GAGGATAACT TCCAATCCGA TTTTTCCAGC CCACGAACCT TCCTTGGTCC 3300
CCGCTCCGGT GCCTTCGAGT CCGATCAATG GGGCCCAAAC GCCTGAAGAT CCAAAGAACC 3360
CTTGTTGAGG TGTATTTCTC GTCTGAGCAA TCTTAGATCC TTCAATTTGC AGTCGCGCAT 3420
ATATACCATC AACATCATCG TCATCACCAT CATTGTCGTC CACAACAGCA CCGCAACGCC 3480
GTTAATGGCA GGGCTTGGAC AACTTGAGGC GGTTTCTAGC AGGTCGGACC GATTGGAGCT 3540
CGACCCAGGG TGCACATCAC CAAGACACAT TCTCCTTCAA ATGAGCGAAC AAGACATAAT 3600
GAGGGAAGTA GTACGCTATC GAACGTCTTC TCACATCCCG GGTTCTTGGC GTATCTTTTG 3660
GCGATTCTTT TTGTTGAAAT AGAAAATTGA AGAGAAAAAA AGAGATCCAC ATGATGAAGA 3720
ACGGCTCTGT AGATTCATGC TCGAAAGAAA GAAAGAAAGA AAAAGAGGGG AACGAACGGA 3780
TCTGAATCTG TGGCCAACCA AAAAGTAGGC ACAAAGATGA CAACAGCGCC CTCTTCGACA 3840
AGTCTTTGAA CTGCTTGTGG ATGAGACAAG TCCCAGCAGA TCAACATTCC TGCTTTACCC 3900
CATGGAGTAT CAAACACCTG AGAATAGGTC TTGCCCGGCT GTAGATAATC TCTGGACCGT 3960
CATATGCGCG AAACGATCAG TACGACCGAC TCTACTCGAA GTCGTCAAGA GCACGGACGA 4020
GAACGAAAAG AGGACAAACC GCTCTGGATG CCATAAATTT CTCTTCTCAT ACCTCTCCCA 4080
CCCACCCTCA GG 4092

(2) INFORMATION FOR SEQ ID NO:6:

(i) SEQUENCE CHARACTERISTICS:
(A) LENGTH: 1091 amino acids
(B) TYPE: amino acid
(D) TOPOLOGY: linear

(ii) MOLECULE TYPE: protein

(iii) HYPOTHETICAL: NO

(vi) ORIGINAL SOURCE:
(A) ORGANISM: Phaffia rhodozyma
(B) STRAIN: ATCC96594

(xi) SEQUENCE DESCRIPTION: SEQ ID NO:6:

Met Tyr Thr Ile Lys His Ser Asn Phe Leu Ser Gln Thr Ile Ser Thr
1               5                   10                  15

Gln Ser Thr Thr Ser Trp Val Val Asp Ala Phe Phe Ser Leu Gly Ser
            20                  25                  30

Arg Tyr Leu Asp Leu Ala Lys Gln Ala Asp Ser Ala Asp Ile Phe Met
        35                  40                  45

Val Leu Leu Gly Tyr Val Leu Met His Gly Thr Phe Val Arg Leu Phe
    50                  55                  60

Leu Asn Phe Arg Arg Met Gly Ala Asn Phe Trp Leu Pro Gly Met Val
65                  70                  75                  80

Leu Val Ser Ser Ser Phe Ala Phe Leu Thr Ala Leu Leu Ala Ala Ser
                85                  90                  95

Ile Leu Asn Val Pro Ile Asp Pro Ile Cys Leu Ser Glu Ala Leu Pro
            100                 105                 110

Phe Leu Val Leu Thr Val Gly Phe Asp Lys Asp Phe Thr Leu Ala Lys
        115                 120                 125

Ser Val Phe Ser Ser Pro Glu Ile Ala Pro Val Met Leu Arg Arg Lys
    130                 135                 140

Pro Val Ile Gln Pro Gly Asp Asp Asp Asp Leu Glu Gln Asp Glu His
145                 150                 155                 160

Ser Arg Val Ala Ala Asn Lys Val Asp Ile Gln Trp Ala Pro Pro Val
                165                 170                 175

Ala Ala Ser Arg Ile Val Ile Gly Ser Val Glu Lys Ile Gly Ser Ser
            180                 185                 190

Ile Val Arg Asp Phe Ala Leu Glu Val Ala Val Leu Leu Leu Gly Ala
        195                 200                 205

Ala Ser Gly Leu Gly Gly Leu Lys Glu Phe Cys Lys Leu Ala Ala Leu
    210                 215                 220

Ile Leu Val Ala Asp Cys Cys Phe Thr Phe Thr Phe Tyr Val Ala Ile
225                 230                 235                 240

Leu Thr Val Met Val Glu Val His Arg Ile Lys Ile Ile Arg Gly Phe
                245                 250                 255

Arg Pro Ala His Asn Asn Arg Thr Pro Asn Thr Val Pro Ser Thr Pro
            260                 265                 270

Thr Ile Asp Gly Gln Ser Thr Asn Arg Ser Gly Ile Ser Ser Gly Pro
        275                 280                 285

Pro Ala Arg Pro Thr Val Pro Val Trp Lys Lys Val Trp Arg Lys Leu
    290                 295                 300

Met Gly Pro Glu Ile Asp Trp Ala Ser Glu Ala Glu Ala Arg Asn Pro
305                 310                 315                 320

Val Pro Lys Leu Lys Leu Leu Leu Ile Leu Ala Phe Leu Ile Leu His
                325                 330                 335

Ile Leu Asn Leu Cys Thr Pro Leu Thr Glu Thr Thr Ala Ile Lys Arg
            340                 345                 350

Ser Ser Ser Ile His Gln Pro Ile Tyr Ala Asp Pro Ala His Pro Ile
        355                 360                 365

Ala Gln Thr Asn Thr Thr Leu His Arg Ala His Ser Leu Val Ile Phe
    370                 375                 380

Asp Gln Phe Leu Ser Asp Trp Thr Thr Ile Val Gly Asp Pro Ile Met
385                 390                 395                 400

Ser Lys Trp Ile Ile Ile Thr Leu Gly Val Ser Ile Leu Leu Asn Gly
                405                 410                 415

Phe Leu Leu Lys Gly Ile Ala Ser Gly Ser Ala Leu Gly Pro Gly Arg
            420                 425                 430

Ala Gly Gly Gly Gly Ala Ala Ala Ala Ala Ala Val Leu Leu Gly Ala
        435                 440                 445

Trp Glu Ile Val Asp Trp Asn Asn Glu Thr Glu Thr Ser Thr Asn Thr
    450                 455                 460

Pro Ala Gly Pro Pro Gly His Lys Asn Gln Asn Val Asn Leu Arg Leu
465                 470                 475                 480

Ser Leu Glu Arg Asp Thr Gly Leu Leu Arg Tyr Gln Arg Glu Gln Ala
                485                 490                 495

Tyr Gln Ala Gln Ser Gln Ile Leu Ala Pro Ile Ser Pro Val Ser Val
            500                 505                 510

Ala Pro Val Val Ser Asn Gly Asn Gly Asn Ala Ser Lys Ser Ile Glu
        515                 520                 525

Lys Pro Met Pro Arg Leu Val Val Pro Asn Gly Pro Arg Ser Leu Pro
    530                 535                 540

Glu Ser Pro Pro Ser Thr Thr Glu Ser Thr Pro Val Asn Lys Val Ile
545                 550                 555                 560

Ile Gly Gly Pro Ser Asp Arg Pro Ala Leu Asp Gly Leu Ala Asn Gly
                565                 570                 575

Asn Gly Ala Val Pro Leu Asp Lys Gln Thr Val Leu Gly Met Arg Ser
            580                 585                 590

Ile Glu Glu Cys Glu Glu Ile Met Lys Ser Gly Leu Gly Pro Tyr Ser
        595                 600                 605

Leu Asn Asp Glu Glu Leu Ile Leu Leu Thr Gln Lys Gly Lys Ile Pro
    610                 615                 620

Pro Tyr Ser Leu Glu Lys Ala Leu Gln Asn Cys Glu Arg Ala Val Lys
625                 630                 635                 640

Ile Arg Arg Ala Val Ile Ser Arg Ala Ser Val Thr Lys Thr Leu Glu
                645                 650                 655

Thr Ser Asp Leu Pro Met Lys Asp Tyr Asp Tyr Ser Lys Val Met Gly
            660                 665                 670

Ala Cys Cys Glu Asn Val Val Gly Tyr Met Pro Leu Pro Val Gly Ile
        675                 680                 685

Ala Gly Pro Leu Asn Ile Asp Gly Glu Val Val Pro Ile Pro Met Ala
    690                 695                 700

Thr Thr Glu Gly Thr Leu Val Ala Ser Thr Ser Arg Gly Cys Lys Ala
705                 710                 715                 720

Leu Asn Ala Gly Gly Gly Val Thr Thr Val Ile Thr Gln Asp Ala Met
                725                 730                 735

Thr Arg Gly Pro Val Val Asp Phe Pro Ser Val Ser Gln Ala Ala Gln
            740                 745                 750

Ala Lys Arg Trp Leu Asp Ser Val Glu Gly Met Glu Val Met Ala Ala
        755                 760                 765

Ser Phe Asn Ser Thr Ser Arg Phe Ala Arg Leu Gln Ser Ile Lys Cys
    770                 775                 780

Gly Met Ala Gly Arg Ser Leu Tyr Ile Arg Leu Ala Thr Ser Thr Gly
785                 790                 795                 800

Asp Ala Met Gly Met Asn Met Ala Gly Lys Gly Thr Glu Lys Ala Leu
                805                 810                 815

Glu Thr Leu Ser Glu Tyr Phe Pro Ser Met Gln Ile Leu Ala Leu Ser
            820                 825                 830

Gly Asn Tyr Cys Ile Asp Lys Lys Pro Ser Ala Ile Asn Trp Ile Glu
        835                 840                 845

Gly Arg Gly Lys Ser Val Val Ala Glu Ser Val Ile Pro Gly Ala Ile
    850                 855                 860

Val Lys Ser Val Leu Lys Thr Thr Val Ala Asp Leu Val Asn Leu Asn
865                 870                 875                 880

Ile Lys Lys Asn Leu Ile Gly Ser Ala Met Ala Gly Ser Ile Gly Gly
                885                 890                 895

Phe Asn Ala His Ala Ser Asp Ile Leu Thr Ser Ile Phe Leu Ala Thr
            900                 905                 910

Gly Gln Asp Pro Ala Gln Asn Val Glu Ser Ser Met Cys Met Thr Leu
        915                 920                 925

Met Glu Ala Val Asn Asp Gly Lys Asp Leu Leu Ile Thr Cys Ser Met
    930                 935                 940

Pro Ala Ile Glu Cys Gly Thr Val Gly Gly Gly Thr Phe Leu Pro Pro
945                 950                 955                 960

Gln Asn Ala Cys Leu Gln Met Leu Gly Val Ala Gly Ala His Pro Asp
                965                 970                 975

Ser Pro Gly His Asn Ala Arg Arg Leu Ala Arg Ile Ile Ala Ala Ser
            980                 985                 990

Val Met Ala Gly Glu Leu Ser Leu Met Ser Ala Leu Ala Ala Gly His
        995                 1000                1005

Leu Ile Lys Ala His Met Lys His Asn Arg Ser Thr Pro Ser Thr Pro
    1010                1015                1020

Leu Pro Val Ser Pro Leu Ala Thr Arg Pro Asn Thr Pro Ser His Arg
1025                1030                1035                1040

Ser Ile Gly Leu Leu Thr Pro Met Thr Ser Ser Ala Ser Val Ala Ser
                1045                1050                1055

Met Phe Ser Gly Phe Gly Ser Pro Ser Thr Ser Ser Leu Lys Thr Val
            1060                1065                1070

Gly Ser Met Ala Cys Val Arg Glu Arg Gly Asp Glu Thr Ser Val Asn
        1075                1080                1085

Val Asp Ala
    1090

(2) INFORMATION FOR SEQ ID NO:7:

    (i) SEQUENCE CHARACTERISTICS:
        (A) LENGTH: 467 amino acids
        (B) TYPE: amino acid
        (D) TOPOLOGY: linear

    (ii) MOLECULE TYPE: protein

    (iii) HYPOTHETICAL: NO

    (vi) ORIGINAL SOURCE:
        (A) ORGANISM: Phaffia rhodozyma
        (B) STRAIN: ATCC96594

    (xi) SEQUENCE DESCRIPTION: SEQ ID NO:7:

Met Tyr Thr Ser Thr Thr Glu Gln Arg Pro Lys Asp Val Gly Ile Leu
1               5                   10                  15

Gly Met Glu Ile Tyr Phe Pro Arg Arg Ala Ile Ala His Lys Asp Leu
            20                  25                  30

Glu Ala Phe Asp Gly Val Pro Ser Gly Lys Tyr Thr Ile Gly Leu Gly
        35                  40                  45

Asn Asn Phe Met Ala Phe Thr Asp Asp Thr Glu Asp Ile Asn Ser Phe
    50                  55                  60

Ala Leu Asn Ala Val Ser Gly Leu Leu Ser Lys Tyr Asn Val Asp Pro
65                  70                  75                  80

Lys Ser Ile Gly Arg Ile Asp Val Gly Thr Glu Ser Ile Ile Asp Lys
                85                  90                  95

Ser Lys Ser Val Lys Thr Val Leu Met Asp Leu Phe Glu Ser His Gly
            100                 105                 110

Asn Thr Asp Ile Glu Gly Ile Asp Ser Lys Asn Ala Cys Tyr Gly Ser
        115                 120                 125

Thr Ala Ala Leu Phe Asn Ala Val Asn Trp Ile Glu Ser Ser Ser Trp
    130                 135                 140

Asp Gly Arg Asn Ala Ile Val Phe Cys Gly Asp Ile Ala Ile Tyr Ala
145                 150                 155                 160

Glu Gly Ala Ala Arg Pro Ala Gly Gly Ala Gly Ala Cys Ala Ile Leu
                165                 170                 175

Ile Gly Pro Asp Ala Pro Val Val Phe Glu Pro Val His Gly Asn Phe
            180                 185                 190

Met Thr Asn Ala Trp Asp Phe Tyr Lys Pro Asn Leu Ser Ser Glu Tyr
        195                 200                 205

Pro Ile Val Asp Gly Pro Leu Ser Val Thr Ser Tyr Val Asn Ala Ile
    210                 215                 220

Asp Lys Ala Tyr Glu Ala Tyr Arg Thr Lys Tyr Ala Lys Arg Phe Gly
225                 230                 235                 240

Gly Pro Lys Thr Asn Gly Val Thr Asn Gly His Thr Glu Val Ala Gly
                245                 250                 255

Val Ser Ala Ala Ser Phe Asp Tyr Leu Leu Phe His Ser Pro Tyr Gly
            260                 265                 270

Lys Gln Val Val Lys Gly His Gly Arg Leu Leu Tyr Asn Asp Phe Arg
        275                 280                 285

Asn Asn Pro Asn Asp Pro Val Phe Ala Glu Val Pro Ala Glu Leu Ala
    290                 295                 300

Thr Leu Asp Met Lys Lys Ser Leu Ser Asp Lys Asn Val Glu Lys Ser
305                 310                 315                 320

Leu Ile Ala Ala Ser Lys Ser Ser Phe Asn Lys Gln Val Glu Pro Gly
                325                 330                 335

Met Thr Thr Val Arg Gln Leu Gly Asn Leu Tyr Thr Ala Ser Leu Phe
            340                 345                 350

Gly Ala Leu Ala Ser Leu Phe Ser Asn Val Pro Gly Asp Glu Leu Val
        355                 360                 365

Gly Lys Arg Ile Ala Leu Tyr Ala Tyr Gly Ser Gly Ala Ala Ala Ser
    370                 375                 380

Phe Tyr Ala Leu Lys Val Lys Ser Ser Thr Ala Phe Ile Ser Glu Lys
385                 390                 395                 400

Leu Asp Leu Asn Asn Arg Leu Ser Asn Met Lys Ile Val Pro Cys Asp
                405                 410                 415

Asp Phe Val Lys Ala Leu Lys Val Arg Glu Glu Thr His Asn Ala Val
            420                 425                 430

Ser Tyr Ser Pro Ile Gly Ser Leu Asp Asp Leu Trp Pro Gly Ser Tyr
        435                 440                 445

Tyr Leu Gly Glu Ile Asp Ser Met Trp Arg Arg Gln Tyr Lys Gln Val
    450                 455                 460

Pro Ser Ala
465

(2) INFORMATION FOR SEQ ID NO:8:

(i) SEQUENCE CHARACTERISTICS:
(A) LENGTH: 432 amino acids
(B) TYPE: amino acid
(D) TOPOLOGY: linear

(ii) MOLECULE TYPE: protein

(iii) HYPOTHETICAL: NO

(vi) ORIGINAL SOURCE:
(A) ORGANISM: Phaffia rhodozyma
(B) STRAIN: ATCC96594

(xi) SEQUENCE DESCRIPTION: SEQ ID NO:8:

Lys Glu Glu Ile Leu Val Ser Ala Pro Gly Lys Val Ile Leu Phe Gly
1               5                   10                  15

Glu His Ala Val Gly His Gly Val Thr Gly Ile Ala Ala Ser Val Asp
            20                  25                  30

Leu Arg Cys Tyr Ala Leu Leu Ser Pro Thr Ala Thr Thr Thr Thr Ser
        35                  40                  45

Ser Ser Leu Ser Ser Thr Asn Ile Thr Ile Ser Leu Thr Asp Leu Asn
    50                  55                  60

Phe Thr Gln Ser Trp Pro Val Asp Ser Leu Pro Trp Ser Leu Ala Pro
65                  70                  75                  80

Asp Trp Thr Glu Ala Ser Ile Pro Glu Ser Leu Cys Pro Thr Leu Leu
                85                  90                  95

Ala Glu Ile Glu Arg Ile Ala Gly Gln Gly Gly Asn Gly Gly Glu Arg
            100                 105                 110

Glu Lys Val Ala Thr Met Ala Phe Leu Tyr Leu Leu Val Leu Leu Ser
        115                 120                 125

Lys Gly Lys Pro Ser Glu Pro Phe Glu Leu Thr Ala Arg Ser Ala Leu
    130                 135                 140

Pro Met Gly Ala Gly Leu Gly Ser Ser Ala Ala Leu Ser Thr Ser Leu
145                 150                 155                 160

Ala Leu Val Phe Leu Leu His Phe Ser His Leu Ser Pro Thr Thr Thr
                165                 170                 175

Gly Arg Glu Ser Thr Ile Pro Thr Ala Asp Thr Glu Val Ile Asp Lys
            180                 185                 190

Trp Ala Phe Leu Ala Glu Lys Val Ile His Gly Asn Pro Ser Gly Ile
        195                 200                 205

Asp Asn Ala Val Ser Thr Arg Gly Gly Ala Val Ala Phe Lys Arg Lys
    210                 215                 220

Ile Glu Gly Lys Gln Glu Gly Gly Met Glu Ala Ile Lys Ser Phe Thr
225                 230                 235                 240

Ser Ile Arg Phe Leu Ile Thr Asp Ser Arg Ile Gly Arg Asp Thr Arg
                245                 250                 255

Ser Leu Val Ala Gly Val Asn Ala Arg Leu Ile Gln Glu Pro Glu Val
            260                 265                 270

Ile Val Pro Leu Leu Glu Ala Ile Gln Gln Ile Ala Asp Glu Ala Ile
        275                 280                 285

Arg Cys Leu Lys Asp Ser Glu Met Glu Arg Ala Val Met Ile Asp Arg
    290                 295                 300

Leu Gln Asn Leu Val Ser Glu Asn His Ala His Leu Ala Ala Leu Gly
305                 310                 315                 320

Val Ser His Pro Ser Leu Glu Glu Ile Ile Arg Ile Gly Ala Asp Lys
                325                 330                 335

Pro Phe Glu Leu Arg Thr Lys Leu Thr Gly Ala Gly Gly Gly Gly Cys
            340                 345                 350

Ala Val Thr Leu Val Pro Asp Asp Phe Ser Thr Glu Thr Leu Gln Ala
        355                 360                 365

Leu Met Glu Thr Leu Val Gln Ser Ser Phe Ala Pro Tyr Ile Ala Arg
    370                 375                 380

Val Gly Gly Ser Gly Val Gly Phe Leu Ser Ser Thr Lys Ala Asp Pro
385                 390                 395                 400

Glu Asp Gly Glu Asn Arg Leu Lys Asp Gly Leu Val Gly Thr Glu Ile
                405                 410                 415

Asp Glu Leu Asp Arg Trp Ala Leu Lys Thr Gly Arg Trp Ser Phe Ala
            420                 425                 430

(2) INFORMATION FOR SEQ ID NO:9:

     (i) SEQUENCE CHARACTERISTICS:
          (A) LENGTH: 401 amino acids
          (B) TYPE: amino acid
          (D) TOPOLOGY: linear

    (ii) MOLECULE TYPE: protein

   (iii) HYPOTHETICAL: NO

    (vi) ORIGINAL SOURCE:
          (A) ORGANISM: Phaffia rhodozyma
          (B) STRAIN: ATCC96594

    (xi) SEQUENCE DESCRIPTION: SEQ ID NO:9:

Met Val His Ile Ala Thr Ala Ser Ala Pro Val Asn Ile Ala Cys Ile
1               5                   10                  15

Lys Tyr Trp Gly Lys Arg Asp Thr Lys Leu Ile Leu Pro Thr Asn Ser
            20                  25                  30

Ser Leu Ser Val Thr Leu Asp Gln Asp His Leu Arg Ser Thr Thr Ser
        35                  40                  45

Ser Ala Cys Asp Ala Ser Phe Glu Lys Asp Arg Leu Trp Leu Asn Gly
    50                  55                  60

Ile Glu Glu Glu Val Lys Ala Gly Gly Arg Leu Asp Val Cys Ile Lys
65                  70                  75                  80

Glu Met Lys Lys Leu Arg Ala Gln Glu Glu Glu Lys Asp Ala Gly Leu
                85                  90                  95

Glu Lys Leu Ser Ser Phe Asn Val His Leu Ala Ser Tyr Asn Asn Phe
            100                 105                 110

Pro Thr Ala Ala Gly Leu Ala Ser Ser Ala Ser Gly Leu Ala Ala Leu
        115                 120                 125

Val Ala Ser Leu Ala Ser Leu Tyr Asn Leu Pro Thr Asn Ala Ser Glu
    130                 135                 140

Leu Ser Leu Ile Ala Arg Gln Gly Ser Gly Ser Ala Cys Arg Ser Leu
145                 150                 155                 160

Phe Gly Gly Phe Val Ala Trp Glu Gln Gly Lys Leu Ser Ser Gly Thr
                165                 170                 175

Asp Ser Phe Ala Val Gln Val Glu Pro Arg Glu His Trp Pro Ser Leu
            180                 185                 190

His Ala Leu Ile Cys Val Val Ser Asp Glu Lys Lys Thr Thr Ala Ser
        195                 200                 205

Thr Ala Gly Met Gln Thr Thr Val Asn Thr Ser Pro Leu Leu Gln His
    210                 215                 220

Arg Ile Glu His Val Val Pro Ala Arg Met Glu Ala Ile Thr Gln Ala
225                 230                 235                 240

Ile Arg Ala Lys Asp Phe Asp Ser Phe Ala Lys Ile Thr Met Lys Asp
                245                 250                 255

Ser Asn Gln Phe His Ala Val Cys Leu Asp Ser Glu Pro Pro Ile Phe
            260                 265                 270

Tyr Leu Asn Asp Val Ser Arg Ser Ile Ile His Leu Val Thr Glu Leu
        275                 280                 285

Asn Arg Val Ser Val Gln Ala Gly Gly Pro Val Leu Ala Ala Tyr Thr
    290                 295                 300

Phe Asp Ala Gly Pro Asn Ala Val Ile Tyr Ala Glu Glu Ser Ser Met
305                 310                 315                 320

Pro Glu Ile Ile Arg Leu Ile Glu Arg Tyr Phe Pro Leu Gly Thr Ala
                325                 330                 335

Phe Glu Asn Pro Phe Gly Val Asn Thr Glu Gly Gly Asp Ala Leu Arg
            340                 345                 350

Glu Gly Phe Asn Gln Asn Val Ala Pro Val Phe Arg Lys Gly Ser Val
        355                 360                 365

Ala Arg Leu Ile His Thr Arg Ile Gly Asp Gly Pro Arg Thr Tyr Gly
    370                 375                 380

Glu Glu Glu Ser Leu Ile Gly Glu Asp Gly Leu Pro Lys Val Val Lys
385                 390                 395                 400

Ala

(2) INFORMATION FOR SEQ ID NO:10:

(i) SEQUENCE CHARACTERISTICS:
(A) LENGTH: 355 amino acids
(B) TYPE: amino acid
(D) TOPOLOGY: linear

(ii) MOLECULE TYPE: protein

(iii) HYPOTHETICAL: NO

(vi) ORIGINAL SOURCE:
(A) ORGANISM: Phaffia rhodozyma
(B) STRAIN: ATCC96594

(xi) SEQUENCE DESCRIPTION: SEQ ID NO:10:

Met Ser Thr Thr Pro Glu Glu Lys Lys Ala Ala Arg Ala Lys Phe Glu
1               5                   10                  15

Ala Val Phe Pro Val Ile Ala Asp Glu Ile Leu Asp Tyr Met Lys Gly
            20                  25                  30

Glu Gly Met Pro Ala Glu Ala Leu Glu Trp Met Asn Lys Asn Leu Tyr
        35                  40                  45

Tyr Asn Thr Pro Gly Gly Lys Leu Asn Arg Gly Leu Ser Val Val Asp
    50                  55                  60

Thr Tyr Ile Leu Leu Ser Pro Ser Gly Lys Asp Ile Ser Glu Glu Glu
65                  70                  75                  80

Tyr Leu Lys Ala Ala Ile Leu Gly Trp Cys Ile Glu Leu Leu Gln Ala
                85                  90                  95

Tyr Phe Leu Val Ala Asp Asp Met Met Asp Ala Ser Ile Thr Arg Arg
            100                 105                 110

Gly Gln Pro Cys Trp Tyr Lys Val Glu Gly Val Ser Asn Ile Ala Ile
        115                 120                 125

Asn Asn Ala Phe Met Leu Glu Gly Ala Ile Tyr Phe Leu Leu Lys Lys
    130                 135                 140

His Phe Arg Lys Gln Ser Tyr Tyr Val Asp Leu Leu Glu Leu Phe His
145                 150                 155                 160

Asp Val Thr Phe Gln Thr Glu Leu Gly Gln Leu Ile Asp Leu Leu Thr
                165                 170                 175

Ala Pro Glu Asp His Val Asp Leu Asp Lys Phe Ser Leu Asn Lys His
            180                 185                 190

His Leu Ile Val Val Tyr Lys Thr Ala Phe Tyr Ser Phe Tyr Leu Pro
        195                 200                 205

Val Ala Leu Ala Met Arg Met Val Gly Val Thr Asp Glu Glu Ala Tyr
    210                 215                 220

Lys Leu Ala Leu Ser Ile Leu Ile Pro Met Gly Glu Tyr Phe Gln Val
225                 230                 235                 240

Gln Asp Asp Val Leu Asp Ala Phe Arg Pro Pro Glu Ile Leu Gly Lys
                245                 250                 255

Ile Gly Thr Asp Ile Leu Asp Asn Lys Cys Ser Trp Pro Ile Asn Leu
            260                 265                 270

Ala Leu Ser Pro Ala Ser Pro Ala Gln Arg Glu Ile Leu Asp Thr Ser
        275                 280                 285

Tyr Gly Gln Lys Asn Ser Glu Ala Glu Ala Arg Val Lys Ala Leu Tyr
    290                 295                 300

Ala Glu Leu Asp Ile Gln Gly Lys Phe Asn Ala Tyr Glu Gln Gln Ser
305                 310                 315                 320

Tyr Glu Ser Leu Asn Lys Leu Ile Asp Ser Ile Asp Glu Glu Lys Ser
                325                 330                 335

Gly Leu Lys Lys Glu Val Phe His Ser Phe Leu Gly Lys Val Tyr Lys
            340                 345                 350

Arg Ser Lys
        355

(2) INFORMATION FOR SEQ ID NO:11:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:11:

GGNAARTAYA CNATHGGNYT NGGNCA 26

(2) INFORMATION FOR SEQ ID NO:12:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:12:

TANARNSWNS WNGTRTACAT RTTNCC 26

(2) INFORMATION FOR SEQ ID NO:13:

(i) SEQUENCE CHARACTERISTICS:
(A) LENGTH: 24 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:13:

GAAGAACCCC ATCAAAAGCC TCGA 24

(2) INFORMATION FOR SEQ ID NO:14:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:14:

AAAAGCCTCG AGATCCTTGT GAGCG 25

(2) INFORMATION FOR SEQ ID NO:15:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:15:

AGAAGCCAGA AGAGAAAA 18

(2) INFORMATION FOR SEQ ID NO:16:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:16:

TCGTCGAGGA AAGTAGAT                                                    18

(2) INFORMATION FOR SEQ ID NO:17:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:17:

GGTACCATAT GTATCCTTCT ACTACCGAAC                                        30

(2) INFORMATION FOR SEQ ID NO:18:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:18:

GCATGCGGAT CCTCAAGCAG AAGGGACCTG                                        30

(2) INFORMATION FOR SEQ ID NO:19:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:19:

GCNTGYTGYG ARAAYGTNAT HGGNTAYATG CC                                     32

(2) INFORMATION FOR SEQ ID NO:20:

(i) SEQUENCE CHARACTERISTICS:
(A) LENGTH: 32 base pairs
(B) TYPE: nucleic acid

(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:20:

ATCCARTTDA TNGCNGCNGG YTTYTTRTCN GT 32

(2) INFORMATION FOR SEQ ID NO:21:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:21:

GGCCATTCCA CACTTGATGC TCTGC 25

(2) INFORMATION FOR SEQ ID NO:22:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:22:

GGCCGATATC TTTATGGTCC T 21

(2) INFORMATION FOR SEQ ID NO:23:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:23:

GGTACCGAAG AAATTATGAA GAGTGG 26

(2) INFORMATION FOR SEQ ID NO:24:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:24:

CTGCAGTCAG GCATCCACGT TCACAC 26

(2) INFORMATION FOR SEQ ID NO:25:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:25:

GCNCCNGGNA ARGTNATHYT NTTYGGNGA 29

(2) INFORMATION FOR SEQ ID NO:26:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:26:

CCCCANGTNS WNACNGCRTT RTCNACNCC 29

(2) INFORMATION FOR SEQ ID NO:27:

(i) SEQUENCE CHARACTERISTICS:
(A) LENGTH: 17 base pairs

(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:27:

ACATGCTGTA GTCCATG 17

(2) INFORMATION FOR SEQ ID NO:28:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:28:

ACTCGGATTC CATGGA 16

(2) INFORMATION FOR SEQ ID NO:29:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(vi) ORIGINAL SOURCE:
(A) ORGANISM: Phaffia rhodozyma
(B) STRAIN: ATCC96594

(xi) SEQUENCE DESCRIPTION: SEQ ID NO:29:

TTGTTGTCGT AGCAGTGGGT GAGAG 25

(2) INFORMATION FOR SEQ ID NO:30:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:30:

GGAAGAGGAA GAGAAAAG 18

(2) INFORMATION FOR SEQ ID NO:31:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:31:

TTGCCGAACT CAATGTAG 18

(2) INFORMATION FOR SEQ ID NO:32:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(vi) ORIGINAL SOURCE:
(A) ORGANISM: Phaffia rhodozyma
(B) STRAIN: ATCC96594

(xi) SEQUENCE DESCRIPTION: SEQ ID NO:32:

GGATCCATGA GAGCCCAAAA AGAAGA 26

(2) INFORMATION FOR SEQ ID NO:33:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(vi) ORIGINAL SOURCE:
(A) ORGANISM: Phaffia rhodozyma
(B) STRAIN: ATCC96594

(xi) SEQUENCE DESCRIPTION: SEQ ID NO:33:

GTCGACTCAA GCAAAAGACC AACGAC 26

(2) INFORMATION FOR SEQ ID NO:34:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:34:

HTNAARTAYT TGGGNAARMG NGA 23

(2) INFORMATION FOR SEQ ID NO:35:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:35:

GCRTTNGGNC CNGCRTCRAA NGTRTANGC 29

(2) INFORMATION FOR SEQ ID NO:36:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:36:

CCGAACTCTC GCTCATCGCC 20

(2) INFORMATION FOR SEQ ID NO:37:

(i) SEQUENCE CHARACTERISTICS:
(A) LENGTH: 20 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:37:

CAGATCAGCG CGTGGAGTGA 20

(2) INFORMATION FOR SEQ ID NO:38:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:38:

CARGCNTAYT TYYTNGTNGC NGAYGA 26

(2) INFORMATION FOR SEQ ID NO:39:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:39:

CAYTTRTTRT CYTGDATRTC NGTNCCDATY TT 32

(2) INFORMATION FOR SEQ ID NO:40:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:40:

ATCCTCATCC CGATGGGTGA ATACT 25

(2) INFORMATION FOR SEQ ID NO:41:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:41:

AGGAGCGGTC AACAGATCGA TGAGC 25

(2) INFORMATION FOR SEQ ID NO:42:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:42:

GAATTCATAT GTCCACTACG CCTGA 25

(2) INFORMATION FOR SEQ ID NO:43:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:43:

GTCGACGGTA CCTATCACTC CCGCC 25